# EUROPEAN PATENT APPLICATION

(11) **EP 1 795 610 A1**
(43) Date of publication of application: **13.06.2007**
(21) Application number: 05026588.3
(22) Date of filing: 06.12.2005
(51) Int. Cl.: C12Q 1/68

(54) **A composition of nucleic acid sequences, specific for inflammatory disease, in particular rheumatoid arthritis**

(71) Applicant: Oligene GmbH, 10117 Berlin (DE)
(72) Inventor: Stuhlmüller, Bruno, 10117 Berlin (DE)
(74) Representative: Engelhard, Markus

(57) **Abstract**

The present invention relates to a composition of nucleic acid sequences specific for inflammatory diseases, in particular rheumatoid arthritis, to a matrix on which such composition has been immobilized and to uses of such composition and/or matrix.

## Description

The present invention relates to a composition of nucleic acid sequences specific for inflammatory diseases, in particular rheumatoid arthritis, to a matrix on which such composition has been immobilized and to uses of such composition and/or matrix.

There is a wealth of inflammatory diseases, which are characterized by a reaction of the body's immune system. Such diseases may contribute to a significant loss of life expectancy and to a loss of well being in the affected individual. Such diseases include, amongst others rheumatoid arthritis, psoriasis, asthma, colitis, multiple sclerosis and systemic lupus erythematosus. In the past, the treatment of such inflammatory diseases has been very unspecific and was based on broad-spectrum imuno suppressive drugs, such as steroids. One disadvantage of such treatment was the considerable number of side effects associated with such steroids. As of more recently, a certain progress has been made in that more specific drugs and agents have been identified that allow the systematic and specific blocking of known targets in the inflammatory reactions involved in these diseases. One of the better approaches of this kind has been a blockage of TNF-α, which had previously been identified as one of the body's signalling molecules allowing the transmittance of information from one cell to another within the body. TNF-α is a member of the so-called cytokine-family. Cytokines, such as TNF-α play an essential role in normal immunity, but in certain diseases, their levels are increased beyond a "normal" level and thus lead to a potentially deleterious reaction. In some of the aforementioned diseases, such as rheumatoid arthritis, use of TNF-α blocking agents has been observed, to some extent, to lead to an improvement in the condition of the respective patent being treated.

Rheumatoid arthritis (RA) causes pain, and swelling, in the lining of a joint and/or internal organs. In other words, it causes an inflammation reaction. This inflammation reaction separates RA from other more common forms of arthritis such as osteoarthritis. RA is a chronic disease, affecting many joints in the body, and results in damage to cartilage, bone, tendons and ligaments. It is presently believed, that the inflammation reaction associated with RA, is triggered by the body's immune system which fails to recognize body tissue as "self", therefore attacks it and leads to a joint damage. Since the immune system's attacking does not stop, the damage deteriorates and ultimately leads to a destruction of cartilage, bone, tendons and ligaments, which, in turn, may lead to a permanent deformity and disability.

It has so far not been possible to ultimately predict whether or not a patient is developing or going to develop RA, at an early stage. Likewise, it has also not been possible to reliably diagnose RA on molecular levels.

Accordingly, it was an object of the present invention to provide for means that allow the reliable diagnosis of RA.

All these objects are solved by a composition comprising at least the following nucleic acid sequences: 213649_at (splicing factor, arginine/serine-rich 7; SEQ ID NO:1), 219607_s_at (membrane-spanning 4-domains, subfamily A, member 4; SEQ ID NO:2), 212179_at (chromosome 6 open reading frame; SEQ ID NO:3), 205239_at (amphiregulin (schwannoma-derived growth factor)) SEQ ID NO:4), 205950_s_at (carbonic anhydrase I) SEQ ID NO:5), 208911_s_at (pyruvate dehydrogenase E-1 beta subunit; SEQ ID NO:6), 211560_s_at (aminolevulinate, delta-, synthase 2; SEQ ID NO:7), 202464_s_at (6-phosphofructo-2-kinase/fructose-2,6-bisphosphatase 3; SEQ ID NO:8), and 212706_at (RAS p21 protein activator 4; SEQ ID NO:9), and/or the complementary counterparts thereof, and/or partial sequences thereof comprising 18 contiguous nucleotides or more and/or the complementary counterparts thereof.

It is to be noted that all "..._at"-identifiers used herein and in the following are Affymetrix identifers, a publicly available database of which has been created by Affymetrix, Inc., USA (see also: NetAffx Analysis Centre: http://www.affymetrix.com/analysis/index.affx).

In one embodiment, said partial sequences of SEQ ID NO: 1-9 are selected from the group comprising SEQ ID NO:20-28 and oligomers of up to 70 nucleotides
which hybridize to SEQ ID NO: 1 in the region from residue no. 131 to residue no. 1,
or hybridize to SEQ ID NO:2 in the region from residue no. 255 to residue no. 33,
or hybridize to SEQ ID NO:3 in the region from residue no. 409 to residue no. 280,
or hybridize to SEQ ID NO:4 in the region from residue no. 310 to residue no. 181,
or hybridize to SEQ ID NO:5 in the region from residue no. 130 to residue no. 1,
or hybridize to SEQ ID NO:6 in the region from residue no. 146 to residue no. 17,
or hybridize to SEQ ID NO:7 in the region from residue no. 1463 to residue no. 1334,
or hybridize to SEQ ID NO:8 from residue no. 136 to residue no. 7,
or hybridize to SEQ ID NO:9 from residue no. 1490 to residue no. 1361. In a preferred embodiment said oligomers are oligomers of 70 nucleotides.

As used herein the phrase "an oligomer which hybridizes to SEQ ID NO: xxx in the region from residue no. [larger number] to residue no. [smaller number]" is meant to signify that the oligomer thus described has the capability of hybridizing to the indicated SEQ ID NO: in the indicated region. The residue numbers in this context refer to the numbering of residues within the indicated SEQ ID NO:xxx. Because the oligomers are reverse complementary the larger number is quoted first and the smaller number is quoted thereafter. The oligomer's capability to hybridize may be due to the fact that the oligomer is fully complementary to the SEQ ID NO:xxx over all the nucleotides of the oligomer, or there may be some mismatches in some residues of the oligomer which, however, do not lead to a disruption of the hybrid between the oligomer and the SEQ ID NO:xxx under the conditons chosen. Preferred hybridization conditions in the context of the present invention are as listed in the examples.

In a preferred embodiment the composition according to the present invention comprises one or more of the following additional sequences and/or the complementary counterparts thereof, and/or partial sequences thereof comprising 18 contiguous nucleotides or more and/or complementary counterparts thereof: 205987_at (CD1C antigen, c polypeptide; SEQ ID NO:10), 214280_x_at (heterogeneous nuclear ribonucleoprotein A1, SEQ ID NO:11), 218204_s_at (FYVE and coiled-coil domain; SEQ ID NO:12), wherein, preferably, said partial sequences of SEQ ID NO:10-12 are selected from the group comprising SEQ ID NO:29-31 and oligomers of up to 70 nucleotides which hybridize to SEQ ID NO: 10 in the region from residue no. 769 to residue no. 640, or hybridize to SEQ ID NO: 11 in the region from residue no. 130 to residue no. 1, or hybridize to SEQ ID NO: 12 in the region from residue no. 130 to residue no. 1.

In one embodiment, the composition according to the present invention furthermore comprises one or more of the following nucleic acid sequences and/or the complementary counterparts thereof, and/or partial sequences thereof comprising 18 nucleotides or more and/or complementary counterparts thereof:
207332_s_at (transferrin receptor (p90, CD71); SEQ ID NO:13), 203115_at (ferrochelatase; SEQ ID NO:14), 219093_at (hypothetical protein FLJ20701; SEQ ID NO:15), 219714_s_at (calcium channel, (voltage-dependent); SEQ ID NO:16), 221622_s_at (uncharacterized hypothalamus protein HT007; SEQ ID NO:17), 221748_s_at (tensin; SEQ ID NO: 18), and 201890_at (ribonucleotide reductase M2 polypeptide; SEQ ID NO:19), wherein, preferably, said partial sequences of SEQ ID NO:13-19 are selected from the group comprising SEQ ID NO:32-38, and oligomers of up to 70 nucleotides
which hybridize to SEQ ID NO:13 in the region from residue no. 192 to residue no. 63,
or hybridize to SEQ ID NO:14 in the region from residue no. 204 to residue no. 75,
or hybridize to SEQ ID NO: 15 in the region from residue no. 226 to residue no. 97,
or hybridize to SEQ ID NO: 16 in the region from residue no. 2225 to residue no. 2096,
or hybridize to SEQ ID NO: 17 in the region from residue no. 130 to residue no. 1,
or hybridize to SEQ ID NO: 18 in the region from residue no. 4834 to residue no. 4765,
or hybridize to SEQ ID NO: 19 in the region from residue no. 349 to residue no. 220.

In one embodiment, said complementary counterparts of said partial sequences comprising 18 contiguous nucleotides or more are 9 sets of primers, each set of primers comprising a forward primer and a reverse primer, each set of primers being able to hybridize to one nucleic acid sequence selected from SEQ ID NO: 1-9, such that different sets of primers are able to hybridize to different nucleic acid sequences and all 9 sets of primers in their entirety are able to hybridize to SEQ ID NO: 1-9, said composition further being suitable to be used in a nucleic acid amplification reaction, preferably a polymerase chain reaction, said primers being selected such that the resulting amplification reaction product(s) are 140-260 nucleotides in length. Suitable primers fulfilling these conditions can be designed by someone skilled in the art, using e.g. software tools, like PRIMER (see below).

Preferably, said 9 sets of primers are SEQ ID NO:39-56.

In one embodiment, the composition furthermore comprises one to three additional sets of primers, each additional set of primers comprising a forward primer and a reverse primer, each additional set of primers being able to hybridize to one nucleic acid sequence selected from SEQ ID NO:10-12, such that different sets of primers are able to hybridize to different nucleic acid sequences, said composition further being suitable to be used in a nucleic acid amplification reaction, preferably a polymerase chain reaction, said primers being selected such that the resulting amplification reaction product(s) are 140-260 nucleotides in length, wherein, preferably, said one to three additional sets of primers are selected from the group comprising SEQ ID NO:57-62.

In one embodiment, said composition furthermore comprises one to seven additional sets of primers, each additional set of primers comprising a forward primer and a reverse primer, each additional set of primers being able to hybridize to one nucleic acid sequence selected from SEQ ID NO: 13-19, such that different sets of primers are able to hybridize to different nucleic acid sequences , said composition further being suitable to be used in a nucleic acid amplification reaction, preferably a polymerase chain reaction, said primers being selected such that the resulting amplification reaction product(s) are 140-260 nucleotides in length, wherein, preferably, said one to seven additional sets of primers are selected from the group comprising SEQ ID NO: 63-76.

In one embodiment, said composition further comprises reagents for nucleic acid amplification and a detectable nucleic acid binding agent. By performing PCR, in particular real-rime-PCR on a patient's sample using the inventive composition, one may determine the original amount and/or expression level of any of the marker genes SEQ ID NO: 1-19.

The objects of the invention are also solved by a solid matrix having a surface on which a nucleic acid composition as defined in any of claims 1-6 or a nucleic acid composition as defined in any of claims 7-13 has been immobilized, wherein, preferably, said matrix is a substrate having a surface, preferably a planar substrate.

In another embodiment, said matrix is a set of beads suitable for use in a high-throughput assay, said beads having surfaces on which said nucleic acid composition as defined in any of claims 1-6 and/or 7-13 has been immobilized. In one embodiment, on said beads a nucleic acid composition as defined in any of claims 1-6 or a nucleic acid composition as defined in any of claims 7-13 has been immobilized. In these embodiments, hybridization events are measured using beads on which nucleic acids, as described, have been immobilized, for capturing target nucleic acids. Such a set of beads can be considered a suspension array system. One commercially available example of such a suspension array system that may be used for the purposes of the present invention is the Bio-Plex® suspension array system of BioRad, as e.g. described in Example 3.

In instances, where the nucleic acid primers as defined in any of claims 7-12 have been immobilized, it is preferred that these serve as capturing nucleic acids for target nucleic acids, to produce hybridization events in case of matching sequences.

The objects of the present invention are also solved by the use of the composition according to the present invention and/or of the matrix according to the present invention for in-vitro-diagnosis, prognosis and/or detection of an inflammatory process, in particular an inflammatory disease, in a patient.

The objects of the present invention are also solved by the use of the composition and the matrix according to the present invention for in-vitro-diagnosis, prognosis and/or detection of an inflammatory process, in particular an inflammatory disease, in a patient, wherein, preferably, said inflammatory process or disease is rheumatoid arthritis, or another chronic inflammatory disease involving monocytes/macrophages.

In one embodiment, the use according to the present invention serves the purpose of monitoring monocyte/macrophage activation in a body fluid or body tissue of an organism.

The objects of the present invention are also solved by an in-vitro method of diagnosing an inflammatory disease, in particular rheumatoid arthritis, in a patient, comprising the steps:
- providing a composition according to any of claims 1-13 and/or a matrix according to any of claims 14-16
- providing a body fluid sample of a patient suspected of having an inflammatory disease preferably rheumatoid arthritis,
- contacting said sample with said composition and/or said matrix,
   - establishing conditions that allow a hybridization reaction to take place between the composition of any of claims 1 - 6 or the composition of any of claims 7-12 or the matrix of any of claims 14 - 16, and nucleic acids present in said sample, or
   - establishing conditions allowing a nucleic acid amplification reaction, preferably PCR, to take place, using the primers of the composition according to any of claims 7-13, on nucleic acids present in said sample,
- determining whether any of SEQ ID NO: 1 - 19 have been specifically upregulated or down-regulated in said sample, by the results of said hybridization or said amplification reaction wherein, preferably, said body fluid is blood, plasma, serum or lymph fluid.

By using a pairwise "comparison expression analysis" (MAS Version 5.0 - Microarray Suite Software of Affymetrix), the present inventors have succeeded in identifying a number of highly specific nucleic acid sequences (SEQ ID NO: 1 - 19) which, in RA in a patient, are specifically either up-regulated (i.e. expression levels higher than in comparative "normal", i.e. healthy subjects) or down-regulated (i.e. expression levels lower than in "normal", i.e. healthy individuals). These specific nucleic acid sequences have so far not been implicated in the pathogenesis of or associated with the symptoms of rheumatoid arthritis and can be used for the diagnosis of RA. In addition thereto, based on SEQ ID NO: 1 - 19 and using the appropriate software tool (PICKY 2.0.0) the present inventors have also identified a number of reverse complementary oligomers (SEQ ID NO: 20 - 38) and additional oligomers centering around SEQ ID NO: 20 - 38 which hybridize to the regions of SEQ ID NO: 1 - 19 and can therefore also be used for the diagnosis of RA.

Additionally, based on SEQ ID NO: 1-19 and using the appropriate software tool (the programme PRIMER from the online software package HUSAR), the present inventors have also identified a number of suitable primers for each of SEQ ID NO: 1-19 which can be used in PCR-reactions, preferably real-time-PCR-reaction, by which the presence of one or several of SEQ ID NO: 1-19 and the quantity thereof can be established, thus also enabling the diagnosis of RA. For each of SEQ ID NO: 1-19, the present inventors have identified a set of primers, wherein each set comprises a forward-primer and a reverse primer. More specifically, these primers, for the purposes of the present application are designated as SEQ ID NO: 39-76.

As used herein, the term "nucleic acid sequence" is meant to designate DNA, RNA or combinations thereof. In a preferred embodiment it refers to DNA.
The term "partial sequence" and "oligomer", as used herein, are used interchangeably.

### Specific description of the invention

In the joints of rheumatoid arthritis (RA) patients, the pannus tissue is characterized by massive infiltration with activated macrophages (Mφ). These cells play a major role in the inflammatory cascade, since they produce a number of pro-inflammatory cytokines (Rooney M, Symons JA, Duff GW.: Interleukin 1 beta in synovial fluid is related to local disease activity in rheumatoid arthritis. Rheumatol Int. 10: 217-219, 1990; Brennan FM, Maini RN, Feldmann M.: TNF alpha--a pivotal role in rheumatoid arthritis? Br. J..Rheumatol.. 31: 293-298, 1992). In addition to tissue Mφ, circulating monocytes (MO) in the peripheral blood of patients with active RA (Hahn G, Stuhlmuller B, Hain N, Kalden JR, Pfizenmaier K, Burmester GR.: Modulation of monocyte activation in patients with rheumatoid arthritis by leukapheresis therapy. J Clin Invest. 1993 Mar;91(3):862-70; Stuhlmuller B, Ungethum U, Scholze S, Martinez L, Backhaus M, Kraetsch HG, Kinne RW, Burmester GR.: Identification of known and novel genes in activated monocytes from patients with rheumatoid arthritis. Arthritis Rheum. 2000 Apr;43(4):775-90; Schulze-Koops H, Davis LS, Kavanaugh AF, Lipsky PE.: Elevated cytokine messenger RNA levels in the peripheral blood of patients with rheumatoid arthritis suggest different degrees of myeloid cell activation. Arthritis Rheum. 1997 Apr;40(4):639-47) and bone marrow precursors of the myelomonocytic lineage are also activated (Thomas R, Lipsky PE.: Dendritic cells: origin and differentiation. Stem Cells. 1996 Mar;14(2):196-206. Papadaki HA, Kritikos HD, Gemetzi C, Koutala H, Marsh JC, Boumpas DT, Eliopoulos GD.: Bone marrow progenitor cell reserve and function and stromal cell function are defective in rheumatoid arthritis: evidence for a tumor necrosis factor alpha-mediated effect. Blood. 2002 Mar 1;99(5):1610-9.Santiago-Schwarz F, Sullivan C, Rappa D, Carsons SE.: Distinct alterations in lineage committed progenitor cells exist in the peripheral blood of patients with rheumatoid arthritis and primary Sjogren's syndrome. J Rheumatol. 1996 Mar;23(3):439-46. Seitz M, Zwicker M, Pichler W, Gerber N.: Activation and differentiation of myelomonocytic cells in rheumatoid arthritis and healthy individuals--evidence for antagonistic in vitro regulation by interferon-gamma and tumor necrosis factor alpha, granulocyte monocyte colony stimulating factor and interleukin 1. J R heumatol. 1992 Jul;19(7):1038-44.), and spontaneously release interleukin-1β (IL-1β), IL-6, tumor necrosis factor-α (TNFα), prostaglandin E2, and neopterin (Seitz M, Deimann W, Gemsa D.: The influence of synovial fluid from patients with rheumatoid arthritis on the proliferation of peripheral blood lymphocytes and the prostanoid release from monocytes. Agents Actions. 1981 Dec;11(6-7):606-8 ; Horneff G, Sack U, Kalden JR, Emmrich F, Burmester GR.: Reduction of monocytemacrophage activation markers upon anti-CD4 treatment. Decreased levels of IL-1, IL-6, neopterin and soluble CD14 in patients with rheumatoid arthritis; Clin Exp Immunol. 1993 Feb;91(2):207-13).. In RA, myeloid precursors are characterized by increased numbers of CD14 molecules (Tomita T, Shimaoka Y, Kashiwagi N, Hashimoto H, Kawamura S, Lee SB, Nakagawa S, Shiho O, Hayashida K, Ochi T.: Enhanced expression of CD14 antigen on myeloid lineage cells derived from the bone marrow of patients with severe rheumatoid arthritis. J Rheumatol. 1997 Mar;24(3):465-9.), and bone marrow stromal cell function are defective probably due to a TNFα-mediated effect (Papadaki et al., Leuk Lymphoma 2002 Apr. 43(4):753-760). Therefore, MO/Mφ play an important role in both local and systemic inflammation of chronic arthritis (Burmester GR, Stuhlmuller B, Keyszer G, Kinne RW.: Mononuclear phagocytes and rheumatoid synovitis: mastermind or workhorse in arthritis? Arthritis Rheum 1997; 40: 5-18.)

During inflammatory processes, various mediators (such as cytokines and chemokines) regulate the recruitment of MO. In patients with RA, circulating MO may have intrinsic phenotypic abnormalities, as evidenced by enhanced expression of Fc-gamma receptor (Burmester GR, Stuhlmuller B, Keyszer G, Kinne RW.: Mononuclear phagocytes and rheumatoid synovitis: mastermind or workhorse in arthritis? Arthritis Rheum 1997; 40: 5-18.) Once in the tissue, MO undergo the poorly-understood process of transformation to Mφ with altered morphology and function (Shiozawa S, Shiozawa K, Fujita T.: Morphologic observations in the early phase of the cartilage-pannus junction. Light and electron microscopic studies of active cellular pannus. Arthritis Rheum. 1983 Apr;26(4):472-8. Kawanaka N, Yamamura M, Aita T, Morita Y, Okamoto A, Kawashima M, Iwahashi M, Ueno A, Ohmoto Y, Makino H.: CD14+,CD16+ blood monocytes and joint inflammation in rheumatoid arthritis. Arthritis Rheum. 2002 Oct;46(10):2578-86.)

In RA, synovial Mφ contribute to joint destruction by differentiating into bone-resorbing osteoclasts (Fujikawa Y, Sabokbar A, N eale S , Athanasou N A.: Human osteoclast formation and bone resorption by monocytes and synovial macrophages in rheumatoid arthritis. Ann Rheum Dis. 1996 Nov;55(11):816-22. Goldring SR.:Bone and joint destruction in rheumatoid arthritis: what is really happening? J Rheumatol Suppl. 2002 Sep;65:44-8. Redlich K, Hayer S, Ricci R, David JP, Tohidast-Akrad M, Kollias G, Steiner G, Smolen JS, Wagner EF, Schett G.: Osteoclasts are essential for TNF-alpha-mediated joint destruction. J C lin Invest. 2002 Nov;110(10):1419-27.) or by releasing cartilage-degrading enzymes (Smeets TJ, Barg EC, Kraan MC, Smith MD, Breedveld FC, Tak PP.: Analysis of the cell infiltrate and expression of proinflammatory cytokines and matrix metalloproteinases in arthroscopic synovial biopsies: comparison with synovial samples from patients with end stage, destructive rheumatoid arthritis. Ann Rheum Dis. 2003 Jul;62(7):635-8. Hansen T, Petrow PK, Gaumann A, Keyszer GM, Eysel P, Eckardt A, Brauer R, Kriegsmann J.: Cathepsin B and its endogenous inhibitor cystatin C in rheumatoid arthritis synovium. J Rheumatol. 2000 Apr;27(4):859-65.) and pro-inflammatory cytokines, e.g., IL-1β and TNFα (Firestein GS, Alvaro-Gracia JM, Maki R, Alvaro-Garcia JM.: Quantitative analysis of cytokine gene expression in rheumatoid arthritis. J Immunol. 1990 May 1;144(9):3347-53. MacNaul KL, Hutchinson NI, Parsons JN, Bayne EK, Tocci MJ: Analysis of IL-1 and TNF-alpha gene expression in human rheumatoid synoviocytes and normal monocytes by in situ hybridization. J Immunol. 1990 Dec 15;145(12):4154-66. Dayer JM, Burger D.: Interleukin-1, tumor necrosis factor and their specific inhibitors. Eur Cytokine Netw. 1994 Nov-Dec;5(6):563-71. Miossec P.: An update on the cytokine network in rheumatoid arthritis. Curr Opin Rheumatol. 2004 May;16(3):218-222.). TNFα is derived primarily from activated MO/Mφ and TNFα promotes the synthesis of other proinflammatory cytokines, stimulates endothelial cells to express adhesion molecules, and accelerates the synthesis of metalloproteinases(Choy EH, Panayi GS.: Cytokine pathways and joint inflammation in rheumatoid arthritis. N Engl J Med. 2001 Mar 22;344(12):907-16.).

Novel microarray strategies are rapidly improving the view on the molecular complexity of chronic diseases and offer more insight into the complexity of gene regulation in RA. Whole-genome microarray approaches are useful to detect the whole gene-transcription levels in cells, and can be specifically aimed at detecting RA-MO relevant or triggered genes,

The possibility of measuring gene expression simultaneously for many thousands of genes represents a challenge in terms of analysis and interpretation. A number of approaches have been proposed and employed in ranking genes according to predictive strength in univariate contexts (Dhanasekaran et al. 2001; Tusher et al. 2001).

In this application, reference is made at various places to sequences SEQ ID NO: 1-19 and 20 - 38, 39-76. The nucleic acid sequences are referred to by their Probe Set ID which more specifically is an entry from the Affymetrix database, publicly available from Affymetrix, Inc., USA

More specifically,
SEQ ID NO:1 is 213649_at (splicing factor, arginine/serine-rich 7; SEQ ID NO:1),
SEQ ID NO:2 is 219607_s_at (membrane-spanning 4-domains, subfamily A, member 4; SEQ ID NO:2),
SEQ ID NO:3 is 212179_at (chromosome 6 open; SEQ ID NO:3),
SEQ ID NO:4 is 205239_at (amphiregulin (schwannoma-derived growth factor)),
SEQ ID NO:5 is 205950_s_at (carbonic anydrase I),
SEQ ID NO:6 is 208911_s_at (pyruvate dehydrogenase E-1 beta subunit),
SEQ ID NO:7 is 211560_s_at (aminolevulinate, delta-, synthase 2),
SEQ ID NO:8 is 202464_s_at (6-phosphofructo-2-kinase/fructose-2,6-bisphosphatase 3),
SEQ ID NO:9 is 212706_at (RAS p21 protein activator 4),
SEQ ID NO:10 is 205987_at (CD1C antigen, c polypeptide),
SEQ ID NO:11 is 214280_x_at (heterogeneous nuclear ribonucleoprotein A1),
SEQ ID NO:12 is 218204_s_at (FYVE and coiled-coil domain),
SEQ ID NO:13 is 207332_s_at (transferrin receptor (p90, CD71),
SEQ ID NO:14 is 203115_at (ferrochelatase protoporphyria);
SEQ ID NO:15 is 219093_at (hypothetical protein FLJ20701),
SEQ ID NO:16 is 219714_s_at (calcium channel, voltage-dependent),
SEQ ID NO:17 is 221622_s_at (uncharacterized hypothalamus protein HT007),
SEQ ID NO: 18 is 221748_s_at (tensin),
SEQ ID NO: 19 is 201890_at (ribonucleotide reductase M2 polypeptide),
and SEQ ID NO: 20 - 38 are the reverse complementary oligomer sequences hybridizing to SEQ ID NO: 1 - 19, respectively.

More specifically,
SEQ ID NO: 20 is a 67-mer reverse complementary and thus hybridizing to the region from residue no. 71 to residue no. 5 of SEQ ID NO: 1.
SEQ ID NO: 21 is a 54-mer reverse complementary and thus hybridizing to the region from residue no. 219 to residue no. 66 of SEQ ID NO: 2.
SEQ ID NO: 22 is a 70-mer reverse complementary and thus hybridizing to the region from residue no. 379 to residue no. 310 of SEQ ID NO: 3.
SEQ ID NO: 23 is a 61-mer reverse complementary and thus hybridizing to the region from residue no. 275 to residue no. 215 of SEQ ID NO: 4.
SEQ ID NO: 24 is a 52-mer reverse complementary and thus hybridizing to the region from residue no. 51 to residue no. 1 of SEQ ID NO: 5.
SEQ ID NO: 25 is a 69-mer reverse complementary and thus hybridizing to the region from residue no. 115 to residue no. 47 of SEQ ID NO: 6.
SEQ ID NO: 26 is a 64-mer reverse complementary and thus hybridizing to the region from residue no. 1430 to residue no. 1367 of SEQ ID NO: 7.
SEQ ID NO: 27 is a 61-mer reverse complementary and thus hybridizing to the region from residue no. 101 to residue no. 41 of SEQ ID NO: 8.
SEQ ID NO: 28 is a 69-mer reverse complementary and thus hybridizing to the region from residue no. 1459 to residue no. 1391 of SEQ ID NO: 9.
SEQ ID NO: 29 is a 68-mer reverse complementary and thus hybridizing to the region from residue no. 738 to residue no. 671 of SEQ ID NO: 10.
SEQ ID NO: 30 is a 64-mer reverse complementary and thus hybridizing to the region from residue no. 66 to residue no. 3 of SEQ ID NO: 11.
SEQ ID NO: 31 is a 70-mer reverse complementary and thus hybridizing to the region from residue no. 92 to residue no. 23 of SEQ ID NO: 12.
SEQ ID NO: 32 is a 55-mer reverse complementary and thus hybridizing to the region from residue no. 154 to residue no. 100 of SEQ ID NO: 13.
SEQ ID NO: 33 is a 66-mer reverse complementary and thus hybridizing to the region from residue no. 172 to residue no. 107 of SEQ ID NO: 14.
SEQ ID NO: 34 is a 65-mer reverse complementary and thus hybridizing to the region from residue no. 193 to residue no. 129 of SEQ ID NO: 15.
SEQ ID NO: 35 is a 51-mer reverse complementary and thus hybridizing to the region from residue no.2185 to residue no. 2135 of SEQ ID NO: 16.
SEQ ID NO: 36 is a 63-mer reverse complementary and thus hybridizing to the region from residue no. 71 to residue no. 9 of SEQ ID NO: 17.
SEQ ID NO: 37 is a 70-mer reverse complementary and thus hybridizing to the region from residue no. 4864 to residue no. 4795 of SEQ ID NO: 18,
SEQ ID NO: 38 is a 68-mer reverse complementary and thus hybridizing to the region from residue no. 318 to residue no. 251 of SEQ ID NO: 19,
SEQ ID NO: 39 and 40 are the preferred forward and reverse primer, respectively for SEQ ID NO: 1,
SEQ ID NO: 41 and 42 are the preferred forward and reverse primer, respectively for SEQ ID NO: 2,
SEQ ID NO: 43 and 44 are the preferred forward and reverse primer, respectively for SEQ ID NO: 3,
SEQ ID NO: 45 and 46 are the preferred forward and reverse primer, respectively for SEQ ID NO: 4,
SEQ ID NO: 47 and 48 are the preferred forward and reverse primer, respectively for SEQ ID NO: 5,
SEQ ID NO: 49 and 50 are the preferred forward and reverse primer, respectively for SEQ ID NO: 6,
SEQ ID NO: 51 and 52 are the preferred forward and reverse primer, respectively for SEQ ID NO: 7,
SEQ ID NO: 53 and 54 are the preferred forward and reverse primer, respectively for SEQ ID NO: 8,
SEQ ID NO: 55 and 56 are the preferred forward and reverse primer, respectively for SEQ ID NO: 9,
SEQ ID NO: 57 and 58 are the preferred forward and reverse primer, respectively for SEQ ID NO: 10,
SEQ ID NO: 59 and 60 are the preferred forward and reverse primer, respectively for SEQ ID NO: 11,
SEQ ID NO: 61 and 62 are the preferred forward and reverse primer, respectively for SEQ ID NO: 12,
SEQ ID NO: 63 and 64 are the preferred forward and reverse primer, respectively for SEQ ID NO: 13,
SEQ ID NO: 65 and 66 are the preferred forward and reverse primer, respectively for SEQ ID NO: 14,
SEQ ID NO: 67 and 68 are the preferred forward and reverse primer, respectively for SEQ ID NO: 15,
SEQ ID NO: 69 and 70 are the preferred forward and reverse primer, respectively for SEQ ID NO: 16,
SEQ ID NO: 71 and 72 are the preferred forward and reverse primer, respectively for SEQ ID NO: 17,
SEQ ID NO: 73 and 74 are the preferred forward and reverse primer, respectively for SEQ ID NO: 18,
SEQ ID NO: 75 and 76 are the preferred forward and reverse primer, respectively for SEQ ID NO: 19,

Furthermore reference is made to the following examples which are given to illustrate, not to limit the present invention.

### Examples

### Example 1

### Patients

Purified monocytes (MO) derived from RA patients prior to treatment and during treatment with the human monoclonal antibody adalimumab were used. Details are summarized as follows: Probes derived from RA patients have been treated with a adalimumab weekly dosage of 20mg or a two weekly dosage of 40mg. Up to 10 mg low dose steroids were given weekly or equally all two weeks. Treatment extended over 2 years. Patients with RA (n=7; all females, age 54 ± 10.7 years (mean ± SD) were classified according to the 1987 revised American College of Rheumatology (ACR) criteria (Hochberg MC, Chang RW, Dwosh I, Lindsey S, Pincus T, Wolfe F.: The American College of Rheumatology 1991 revised criteria for the classification of global functional status in rheumatoid arthritis. Arthritis Rheum. 35: 498-502, 1992) and underwent treatment with adalimumab (20 mg per week or 40 mg every 2 weeks in the outpatient Rheumatology Clinic of the Charité University Hospital in Berlin, Germany. Patients underwent clinical and laboratory investigations.

Peripheral blood (30 to 35 ml) was obtained by venipuncture, immediately stored in heparin-containing vacutainers (Beckton-Dickinson, Rutherford, NJ, USA), and cooled to 4°C. Blood samples were subjected to a Ficoll-Hypaque gradient (d=1.077 g/ml; Biochrom, Berlin, Germany). To enrich MO, negative selection magnetic cell sorting (MACS; Miltenyi; Bergisch Gladbach, Germany) was subsequently applied. Purification of MO was spot tested by FACS analysis using CD14 and CD45 antibodies (Beckman-Coulter, Krefeld, Germany) addicting MO purities of (83-90%). Performing viability tests, 98% of purified MO were vital using propidiumiodide staining (Pharmingen, San-Diego, CA, USA). All steps of MO preparation were performed at 4°C. After purification MO, cells were lyzed in 4M RLT-buffer and RNA was purified using the RNeasy mini elute kit (Quiagen, Düsselforf, Germany). Quantification and quality control of RNA was performed in a Bioanalyzer 2100 unit (Agilent, Palo Alto, USA) at 260/280nm.

### RNA amplification and labeling

For target synthesis starting from 500ng of total RNA, an initial round of amplification w as performed before synthesis of biotin-labeled cRNA. For the first round of amplification, synthesis of first- and second-strand cDNA was performed using the standard protocol provided by the manufacturer (Affymetrix, USA). Instead of proceeding to use the double-stranded cDNA in the biotin-labeled *in vitro* transcription reaction, the cDNA was resuspended in 8 µl of RNase-free water and used as a template to transcribe unlabeled antisense RNA (aRNA) using T7-RNA polymerase and the Megascript kit (Ambion, Cambridgeshire, UK). The reaction was incubated for 4 hours at 37°C and the resulting aRNA was purified using RNeasy spin columns (Quiagen; Hilden, Germany). Eluted aRNA was precipitated by adding 0.1 volume of 7.5 mol/L ammonium acetate, 0.02 volumes of 5 mg/ml linear acrylamide (Ambion; Huntingdon, UK), and 2.5 volumes of 100% ethanol, and resuspended in 10 µl of RNase-free water. A second round of amplification was initiated by using the aRNA as template. After annealing aRNA with 0.7 µmol/L random hexamers (TibMolBiol; Berlin, Germany) for 10 minutes at 70°C, the mixture was chilled on ice and extended in a 20-µl reaction containing 4 µl of 5x first-strand reaction buffer, 2 µl of 0.1 mol/L dithiothreitol, 1 µl of 10 mmol/L dNTPs, and 1 µl of Superscript II (Stratagene; Heidelberg, Germany). After a 1-hour incubation at 42°C, 1 µl of 2 U/ml of RNase H was added, incubated for 20 minutes at 37°C, and inactivated at 95°C for 5 minutes. The resulting first-strand cDNA was annealed to 100 pmol of high performance liquid chromotography (HPLC)-purified T7T₂₄ primer (Tib Molbiol; Berlin, Germany) for 10 minutes at 70°C. Then, second-strand cDNA synthesis was performed by adding 90 µl of RNase-free water, 30 µl of 5x second-strand reaction buffer, 3 µl of 10 mmol/L dNTPs, 10 U DNA ligase, 40 U DNA polymerase, and 2 U of RNase H. After incubating the second-strand cDNA reaction for 2 hours at 16°C, 20 U of T4 DNA polymerase were added, followed by incubation at 16°C for 10 minutes. The second-strand cDNA synthesis was stopped by adding 10 µl of 0.5 mol/L ethylenediaminetetraacetic acid. Double-stranded cDNA was purified by phenol:chloroform:isoamyl alcohol extraction using phase-lock-gel (Eppendorf; Hamburg, Germany), precipitated with 0.5 volumes of 7.5 mol/L ammonium acetate, 2 µg of glycogen, and 2.5 volumes of 100% ethanol, and resuspended in 22 µl of RNase-free water (Ambion).

### Labelling of cRNA and Gene-Chip Hybridization

Biotinylated or aminoallyl-labelled cRNA target was generated from both amplified cRNAs using the Bioarray high-yield transcription kit (Enzo, New York, NY) or the aminoallyl-label kit (Ambion, UK) following the manufacturer's protocols. After a 5-hour incubation at 37°C, the final biotin-labelled cRNA product was purified using RNeasy spin columns (Qiagen) and eluted in 40 µl of RNase-free water. The concentration of biotin-labeled cRNA was determined by UV absorbance and was analyzed for quality using the bioanalyzer 2100 (Agilent). In a 11 cases, 15ug of each biotinylated c RNA preparation was fragmented, assessed by g el electrophoresis, and placed in a hybridization cocktail containing four biotinylated hybridization controls (BioB, BioC, BioD, and Cre) as recommended by the manufacturer (Affymetrix). Samples were fragmented to 200 nucleotides or less, heated at 99°C for 5 min and hybridized for 16 h at 45°C to Hg_U133A microarrays. The microarrays and stained with streptavidin-phycoerythrin. Samples were hybridized to Affymetrix test and HG-U133A GeneChip arrays for 16 hours. GeneChips were washed at low (6X SSPE) and high (100mM MES, 0.1M NaCl) stringency and stained using the instrument's standard Eukaryotic GE Wash 2' protocol, using antibody-mediated signal amplification according to the instructions of the manufacturer (Affymetrix). Scanning of test and HgU133 arrays was performed in an GeneArray 2500A (Agilent, Palo Alto, CA, USA) at 3um resolution and an excitation at 570 nm.

### Data Analysis

The microarray images from the scanned chips were processed using the Affymetrix standard scanning Microarray Analysis Suite 5.0 (MAS 5.0). In the first set of validation experiments the image from each GeneChip was individually scaled such that the average intensity value for all arrays and was adjusted to scaling factor 150. Scaled average difference value, log average ratio, and absolute call data from each chip were exported to flat text files and used for numerical analysis. For analysis of clinical specimens, chip images were normalized to the healthy donor samples, across all probe pair sets. Difference call, fold change, average difference value, and absolute call data from each of the three specimen pairs analyzed were exported to flat text files. SOM clustering was performed using the Affymetrix DMT software.

Hierarchical clustering and PAM analysis was performed using the Affymetrix MAS 5.0 or the Genesis software solution (http://genome.tugraz.at/Software/ GenesisCenter.html).

### Normalization of array data

Normalization by correction of technical variation among individual microarray hybridizations was conducted using a two-step normalization procedure described in detail elsewhere (Dozmorov I, Centola M.: An associative analysis of gene expression array data.Bioinformatics. 2003 Jan 22;19(2):204-11.). This procedure is based on the fact that spot intensities from genes not expressed by the samples of interest constitute noise and are therefore normally distributed. The method models the signals from non-expressed genes to a normal distribution with a mean of 0 and standard deviation (SD) of 1, using an iterative nonlinear curve-fitting procedure.

### Analysis of differentially expressed genes in the RA patient and ND groups

These analyses are performed using standard statistical analysis methods in MAS 5.0 software and include:
1. Selection of statistically different levels of expression using the Student's *t*-test with the commonly accepted significance threshold of *P* < 0.05. Because of the large number of genes present on microarrays, a significant proportion of genes identified as differentially expressed in this manner will be false positive determinations at this threshold level.
2. An associative *t*-test, in which the replicated residuals for each gene in the experimental group are compared with the entire set of residuals from the reference group (defined above). The hypothesis t hat gene expression in the experimental group, p resented as replicated r e-siduals (deviations from averaged control-group profile), is distributed similarly to the several thousand members of the normally distributed set of residuals for gene expressions in the reference group is tested. The significance threshold is corrected to 1/(number of genes) to make it improbable that false positives arise. Only genes with P-values below the threshold of both the Student's *t*-test and the associative *t*-test are then presented in tables as differentially expressed genes. Relative ratios of expression for genes that are differentially expressed above background in both groups are calculated.
3. Genes expressed distinctively above background in one group and not in another are defined as uniquely expressed genes.

### Example 2

### Results:

### Patients, study details, clinical and laboratory assessments, and normal donors:

Purified MO derived from RA patients selected from the Abbott DE011 clinical trial study were used. Gene expression analyses were perfomed using MO from 7 RA-patient MO's prior to and during anti-TNFα treatment and compared to the MO gene expression from 7 normal donors. ACR50 criteria and EULAR defined ACR28 criteria based on the 28 joint count modified disease activity score (Prevoo ML, van 't Hof MA, Kuper HH, van Leeuwen MA, van de Putte LB, van Riel PL.: Modified disease activity scores that include twenty-eight-joint counts. Development and validation in a prospective longitudinal study of patients with rheumatoid arthritis. Arthritis Rheum. 1995,38: 44-48.van Gestel AM, Prevoo ML, van 't Hof MA, van Rijswijk MH, van de Putte LB, van Riel PL.: Development and validation of the European League Against Rheumatism response criteria for rheumatoid arthritis. Comparison with the preliminary American College of Rheumatology and the World Health Organization/International League Against Rheumatism Criteria. Arthritis Rheum. 1996 Jan;39(1):34-40.) were calculated. All 7 RA-patients were rheumatoid factor positive. There was no significant correlation of RF and anti-nuclear autoantibody (ANA) titers.

### MO separation and RNA preparation

Patient and normal donor blood samples (25-40ml) were obtained by venopucture after patient clinical sessions and cooled on ice immediately. Peripheral blood mononuclear cells (PBMC) were enriched by ficoll hypaque (Biocoll; Biochrom, Berlin, Germany) density gradient centrifugation. MO preparation included equally 7 samples from normals, RA patients prior to and during adalulimab treatment. Untouched MO were purified using negative magentic cell sorting (MACS) and 1.7-4.2 x10⁶ MO with purity ranges from 86-90% tested by FACS analysis using a anti-CD14 / anti-CD45 antibody mix were obtained. Performing viability tests, 98% of purified MO were vital using propidiumiodide staining. Approximately 1.5-3.2ug total MO-RNA from age matched female ND and RA-patients.

### Affymetrix gene expression profiling and analysis

Differentially gene expression in MO from normals (n=7) (i.e. healthy humans not affected by RA) and from real RA patients (n=7) was perfomed using genome wide Affymetrix HG-U133A screening representative of ~22,000 genes. RNA extracted from these cases was converted into labeled cRNA and hybridized to the microarrays. After normalization using MAS v5.0 hierarchical clustering analysis using the Genesis WWW platform software (Sturn A, Quackenbush J, Trajanoski Z.: Genesis: cluster analysis of microarray data. Bioinformatics 2002 Jan;18(1):207-8) was performed.

Expression profiling of MO from patients with RA (n=7) and ND (n=7; Signal log ratio (SLR) ≥1; 21 experiments or mean fold expression revealed differentially expressed genes as defined by group-specific regulation in ≥80% of pairwise "comparison expression analysis" MAS v 5.0. Results were obtained by request to data bank established in FileMaker 6.0.

Using this method the following genes, summarized in tables 1 and 2, could be identified as being specifically upregulated or downregulated in comparison with healthy "normal" individuals.

For explanation "SLR" means "signal log ratio", i.e. SLR of 1 means a 2 fold change expression and a SLR of 3.17 means a 9.02 fold change in comparison between two."Mean_Chg_fold_ind" and "Mean_Chg_fold decr" means "mean change fold induced" and "mean change fold decreased";

"MeanSLR_ind" and "MeanSLR_decr" means "mean SLR induced" and "decreased" respectively.

"%_Chg_ind" and "%_Chg_decr" mean "percent change induced" and "percent change decreased".

**Table 1A: Increased and decreased expressed genes in RA vs. ND**

| Increased expressed genes in Rheumatoid arthritis (RA) patients vs. Normal donors (ND) | | | | | | |
|---|---|---|---|---|---|---|
| Gene_ID | %_Chg_ind | %_Chg_decr | Mean_Chg_fold ind | Mean_Chg_fold decr | Mean SLR_ind | Mean_SLR_decr |
| 219607_s_at | 91.84 | 0 | 3.26 | -1 | 1,7 | 0 |
| 205239_at | 81.63 | 4.08 | 9.61 | -2.3 | 3.27 | -1.20 |
| 205950_s_at | 81.63 | 4.08 | 9.02 | -1.8 | 3.17 | -0.85 |
| 211560_s_at | 87.76 | 4.08 | 39.52 | -2.3 | 5.3 | -1.2 |
| 202464_s_at | 81.63 | 0 | 3.44 | -1 | 1.78 | 0 |

| Decreased expressed genes in Rheumatoid arthritis (RA) patients vs. Normal donors (ND) | | | | | | |
|---|---|---|---|---|---|---|
| Gene_ID | %_Chg_ind | %_Chg_decr | Mean_Chg_foid ind | Means_Chg_fold decr | Mean SLR_ind | Mean_SLR_decr |
| 213649_s_at | 0 | 100 | 1 | -3.07 | 0 | -1.62 |
| 212179_at | 2.04 | 87.76 | 1.74 | -2.35 | 0.8 | -1.23 |
| 208911_s_at | 0 | 83.67 | 1 | -1.64 | 0 | -1.72 |
| 212706_at | 0 | 83.67 | 1 | -4.87 | 0 | -2.28 |

**Table 1B: Increased and decreased expressed genes in RA vs. ND**

| Increased expressed genes in Rheumatoid arthritis (RA) patients vs. Normal donors (ND) | | | | | | |
|---|---|---|---|---|---|---|
| Gene_ID | %_Chg_ind | %_Chg_decr | Mean_Chg_fold ind | Means_Chg_fold decr | Mean SLR_ind | Mean_SLR_decr |
| 207332_s_at | 81.63 | 2.04 | 2.88 | -1.62 | 1.53 | -0.70 |
| 203115_at | 83.67 | 8.16 | 5.4 | -2.03 | 2.43 | -1.03 |
| 221748_s_at | 81.63 | 4.08 | 6.23 | -2.55 | 2.64 | -1.35 |
| 201890_at | 81.63 | 8.16 | 6.64 | -2.25 | 2.73 | -1.18 |

| Decreased expressed genes in Rheumatoid arthritis (RA) patients vs. Normal donors (ND) | | | | | | |
|---|---|---|---|---|---|---|
| Gene_ID | %_Chg_ind | %_Chg_decr | Mean_Chg_fold ind | Means_Chg_fold decr | Mean SLR_ind | Mean_SLR_decr |
| 205987_at | 0 | 87.76 | 1 | -3.4 | 0 | -1.77 |
| 214280_x_at | 0 | 81.63 | 1 | -1.97 | 0 | -0.98 |
| 218204_s_at | 0 | 85.71 | 1 | -2.02 | 0 | -1.01 |
| 219093_at | 0 | 85.71 | 1 | -4.05 | 0 | -2.02 |
| 219714_s_at | 0 | 83.67 | 1 | -2.77 | 0 | -1.47 |
| 221622 | 0 | 85.71 | 1 | -1.80 | 0 | -0.90 |

**Table 2A: Gene descriptions**

| | |
|---|---|
| 213649_at | Consensus includes gb:AA524053 /FEA=EST /DB_XREF=gi:2264981 /DB_XREF=est:ng33b07.s1 /CLONE=IMAGE:936565 /UG=Hs.184167 splicing factor, arginineserine-rich 7 (35kD) |
| 219607_s_at | gb:NM_024021.1 /DEF=Homo sapiens membrane-spanning 4-domains, subfamily A, member 4 (MS4A4), mRNA. /FEA=mRNA /GEN=MS4A4 /PROD=membrane-spanning 4-domains, subfamily A, member4 /DB_XREF=gi:13430865 /UG=Hs.325960 membrane-spanning 4-domains, subfamily A, member 4 /FL=gb:AB013102.1 gb:NM_024021.1 gb:AF068288.1 gb:NM_016650.1 |
| 212179_at | Consensus includes gb:AW157501 /FEA=EST/DB_XREF=gi:6228902 /DB_XREF=est:au83a02.x1 /CLONE=)MAGE:2782826 /UG=Hs.18368 DKFZP564B0769 protein |
| 205239_at | gb:NM_001657.1 /DEF=Homo sapiens amphiregulin (schwannoma-derived growth factor) (AREG), mRNA. /FEA=mRNA /GEN=AREG /PROD=amphiregulin (schwannoma-derived growth factor) /DB_XREF=gi:4502198 /UG=Hs.270833 amphiregulin (schwannoma-derived growth factor) /FL=gb:M30704.1 gb:NM_001657.1 |
| 205950_s_at | gb:NM_001738.1 /DEF=Homo sapiens carbonic anhydrase I(CA1), mRNA. /FEA=mRNA/GEN=CA1 /PROD=carbonic anhydrase I /DB_XREF=gi:4502516 /UG=Hs.23118 carbonic anhydrase I/FL=gb:M33987.1 gb:NM_001738.1 |
| 208911_s_at | gb:M34055.1 /DEF=Human pyruvate dehydrogenase E1-beta subunit mRNA, complete cds. /FEA=mRNA /GEN=PDHB /DB_XREF=gi:190791 /UG=Hs.979 pyruvate dehydrogenase (lipoamide) beta /FL=gb:BC000439.1 gb:BC001924.1 gb:J03576.1 gb:M34479.1 gb:M54788.1 gb:M34055.1 gb:NM_000925.1 |
| 211560_s_at | gb:AF130113.1 /DEF=Homo sapiens clone FLB8929 PRO2399 mRNA, complete cds. /FEA=mRNA/PROD=PRO2399 /DB_XREF=gi:11493529 /UG=Hs.79103 cytochrome b5 outer mitochondrial membrane precursor /FL=gb:AF130113.1 |
| 202464_s_at | gb:NM_004566.1 /DEF=Homo sapiens 6-phosphofructo-2-kinasefructose-2,6-biphosphatase 3 (PFKFB3), mRNA. /FEA=mRNA /GEN=PFKFB3 /PROD=6-phosphofructo-2-kinasefructose-2,6-biphosphatase 3 /DB_XREF=gi:4758899/UG=Hs.195471 6-phosphofructo-2-kinasefructose-2,6-biphosphatase 3/FL=gb:D49817.1 gb:AF109735.1 gb:NM_004566.1 |
| 212706_at | Consensus includes gb:AB011110.2/DEF=Homo sapiens mRNA for KIAA0538 protein, partial cds. /FEA=mRNA /GEN=KIAA0538 /PROD=KIAA0538 protein /DB_XREF=gi:6635196 /UG=Hs.184367 GTPase activating protein-like |

**Table 2B: Gene descriptions**

| | |
|---|---|
| 205987_at | gb:NM_001765.1 /DEF=Homo sapiens CD1C antigen, c polypeptide (CD1C), mRNA. /FEA=mRNA/GEN=CD1C/PROD=CD1C antigen, c polypeptide /DB_XREF=gi:4502646 /UG=Hs.1311 CD1C antigen, c polypeptide /FL=gb:M28827.1 gb:NM_001765.1 |
| 214280_x_at | Consensus includes gb:X79536.1 /DEF=H.sapiens mRNA for hnRNPcore protein A1. /FEA=mRNA /PROD=hnRNPcore protein A1 /DB_XREF=gi:496897 /UG=Hs.249495 heterogeneous nuclear ribonucleoprotein A1 |
| 218204_s_at | gb:NM_024513.1 /DEF=Homo sapiens FYVE and coiled-coil domain containing 1 (FYCO1), mRNA. /FEA=mRNA/GEN=FYCO1 /PROD=FYVE and coiled-coil domain containing 1 /DB_XREF=gi:13470091 /UG=Hs.257267 FYVE and coiled-coil domain containing 1 /FL=gb:NM_024513.1 |
| 207332_s_at | gb:NM_003234.1 /DEF=Homo sapiens transferrin receptor (p90, CD71) (TFRC), mRNA. /FEA=mRNA /GEN=TFRC /PROD=transferrin receptor (p90, CD71) /DB_XREF=gi:4507456 /UG=Hs.77356 transferrin receptor (p90, CD71) /FL=gb:NM_003234.1 |
| 203115_at | Consensus includes gb:AU152635 /FEA=EST /DB_XREF=gi:11014156 /DB_XREF=est:AU152635 /CLONE=NT2RP3001344 /UG=Hs.26 ferrochelatase (protoporphyria) /FL=gb:NM_000140.1 |
| 219093_at | gb:NM_017933.1 /DEF=Homo sapiens hypothetical protein FLJ20701 (FLJ20701), mRNA. /FEA=mRNA /GEN=FLJ20701 /PROD=hypothetical protein FLJ20701 /DB_XREF=gi:8923631 /UG=Hs.169764 hypothetical protein FLJ20701 /FL=gb:NM_017933.1 |
| 219714_s_at | gb:NM_018398.1 /DEF=Homo sapiens calcium channel alpha2-delta3 subunit (HSA272268), mRNA. /FEA=mRNA /GEN=HSA272268 /PROD=calcium channel alpha2-delta3 subunit /DB_XREF=gi:8923764 /UG=Hs.22958 calcium channel, voltage-dependent, alpha 2delta 3 subunit /FL=gb:NM_018398.1 |
| 221622_s_at | gb:AF246240.1 /DEF=Homo sapiens HT026 mRNA, complete cds. /FEA=mRNA /PROD=HT026 /DB_XREF=gi:12005514 /UG=Hs.24371 uncharacterized hypothalamus protein HT007 /FL=gb:AF246240.1 |
| 221748_s_at | Consensus includes gb:AL046979 /FEA=EST /DB_XREF=gi:5435035 /DB_XREF=est:DKFZp586K0617_s1 /CLONE=DKFZp586K0617 /UG=Hs.9973 tensin |
| 201890_at | Consensus includes gb:BE966236 /FEA=EST /DB_XREF=gi:11771437 /DB_XREF=est:601660172R1 /CLONE=IMAGE:3905920 /UG=Hs.75319 ribonucleotide reductase M2 polypeptide /FL=gb:NM_001034.1 |

None of these sequences according to the present invention have so far been implicated in RA. and, in any case, show a % change induced or decreased > 81, which means that in > 81% of RA-patients, these genes are induced or decreased in terms of their expression, a diagnosis accuracy not achieved so far. This is far better than any of the standard means for diagnosis. To ensure the diagnosis of RA especially in conventional diagnosis several patient visits over a time period of minimal half a year will be needed. Therefore, this method is useful to ensure the diagnosis of RA and furthermore the quantitative gene expression levels will also be useful in relation to a individual successful therapy. This will be of particular interest especially if RA is at an early stage, i.e. before an onset of the disease manifests itself in clinically detectable symptoms.

Furthermore, starting from these sequences of tables 1 and 2, a number of cDNA sequences were identified using the software package "HUSAR (http://genius.embnet.dkfz-heidelberg.de/menu/w2h/w2hdkfz/Onlinezugang (http://genius.embnet.dkfz-heidelberg.de/menu/w2h/w2hdkfz/). The cDNA sequences were read in advanced mode via mode "FETCH" into the inventors' own database using the command "refseq_DNA:Acc.#". Then they were converted from GCG format into FASTA format using the subprogram "Utilities/Export-Import/ ToFastA". The oligo design of the FASTA-sequences was performed with the reverse complementary sequences using the software "PICKY 2.0.0" (http://www.complex.iastate.edu/download/Picky/index.html) using the predefined standard conditions.

Using this method, oligomers SEQ ID NO:20-38 could be identified which are reverse complementary to stretches of SEQ ID NO:1 - 19. These oligomers are also useful in the reliable and early diagnosis of RA. Because the sequences were tested for differential splicing forms in the mRNA populations using the BLASTn software package of "HUSAR" these sequences as targets will be comparable with cDNA sequences.

More specifically, SEQ ID NO: 20 is a 67-mer reverse complementary and thus hybridizing to the region from residue no. 71 to residue no. 5 of SEQ ID NO: 1.
SEQ ID NO: 21 is a 54-mer reverse complementary and thus hybridizing to the region from residue no. 219 to residue no. 66 of SEQ ID NO: 2.
SEQ ID NO: 22 is a 70-mer reverse complementary and thus hybridizing to the region from residue no. 379 to residue no. 310 of SEQ ID NO: 3.
SEQ ID NO: 23 is a 61-mer reverse complementary and thus hybridizing to the region from residue no. 275 to residue no. 215 of SEQ ID NO: 4.
SEQ ID NO: 24 is a 52-mer reverse complementary and thus hybridizing to the region from residue no. 51 to residue no. 1 of SEQ ID NO: 5.
SEQ ID NO: 25 is a 69-mer reverse complementary and thus hybridizing to the region from residue no. 115 to residue no. 47 of SEQ ID NO: 6.
SEQ ID NO: 26 is a 64-mer reverse complementary and thus hybridizing to the region from residue no. 1430 to residue no. 1367 of SEQ ID NO: 7.
SEQ ID NO: 27 is a 61-mer reverse complementary and thus hybridizing to the region from residue no. 101 to residue no. 41 of SEQ ID NO: 8.
SEQ ID NO: 28 is a 69-mer reverse complementary and thus hybridizing to the region from residue no. 1459 to residue no. 1391 of SEQ ID NO: 9.
SEQ ID NO: 29 is a 68-mer reverse complementary and thus hybridizing to the region from residue no. 738 to residue no. 671 of SEQ ID NO: 10.
SEQ ID NO: 30 is a 64-mer reverse complementary and thus hybridizing to the region from residue no. 66 to residue no. 3 of SEQ ID NO: 11.
SEQ ID NO: 31 is a 70-mer reverse complementary and thus hybridizing to the region from residue no. 92 to residue no. 23 of SEQ ID NO: 12.
SEQ ID NO: 32 is a 55-mer reverse complementary and thus hybridizing to the region from residue no. 154 to residue no. 100 of SEQ ID NO: 13.
SEQ ID NO: 33 is a 66-mer reverse complementary and thus hybridizing to the region from residue no. 172 to residue no. 107 of SEQ ID NO: 14.
SEQ ID NO: 34 is a 65-mer reverse complementary and thus hybridizing to the region from residue no. 193 to residue no. 129 of SEQ ID NO: 15.
SEQ ID NO: 35 is a 51-mer reverse complementary and thus hybridizing to the region from residue no.2185 to residue no. 2135 of SEQ ID NO: 16.
SEQ ID NO: 36 is a 63-mer reverse complementary and thus hybridizing to the region from residue no. 71 to residue no. 9 of SEQ ID NO: 17.
SEQ ID NO: 37 is a 70-mer reverse complementary and thus hybridizing to the region from residue no. 4864 to residue no. 4795 of SEQ ID NO: 18, and
SEQ ID NO: 38 is a 68-mer reverse complementary and thus hybridizing to the region from residue no. 318 to residue no. 251 of SEQ ID NO: 19.

Furthermore in addition to these SEQ ID NO: 20 - 38, a number of oligomers with up to 70 nucleotides have been identifed which are centered around and complementary to the regions of SEQ ID NO:1 - 19 to which regions the oligomers SEQ ID NO:20 - 38 bind. It can be reasonably assumed that these additional oligomers also are useful in the diagnosis of RA with high reliablility.
More specifically, these oligomers are oligomers of up to 70 nucleotides, preferably of precisely 70 nucleotides
which hybridize to SEQ ID NO:1 in the region from residue no. 131 to residue no. 1,
or hybridize to SEQ ID NO:2 in the region from residue no. 255 to residue no. 33,
or hybridize to SEQ ID NO:3 in the region from residue no. 409 to residue no. 280,
or hybridize to SEQ ID NO:4 in the region from residue no. 310 to residue no. 181,
or hybridize to SEQ ID NO:5 in the region from residue no. 130 to residue no. 1,
or hybridize to SEQ ID NO:6 in the region from residue no. 146 to residue no. 17,
or hybridize to SEQ ID NO:7 in the region from residue no. 1463 to residue no. 1334,
or hybridize to SEQ ID NO:8 from residue no. 136 to residue no. 7,
or hybridize to SEQ ID NO:9 from residue no. 1490 to residue no. 1361,
or hybridize to SEQ ID NO: 10 in the region from residue no. 769 to residue no. 640,
or hybridize to SEQ ID NO: 11 in the region from residue no. 130 to residue no. 1,
or hybridize to SEQ ID NO:12 in the region from residue no. 130 to residue no. 1,
or hybridize to SEQ ID NO:13 in the region from residue no. 192 to residue no. 63,
or hybridize to SEQ ID NO:14 in the region from residue no. 204 to residue no. 75,
or hybridize to SEQ ID NO:15 in the region from residue no. 226 to residue no. 97,
or hybridize to SEQ ID NO:16 in the region from residue no. 2225 to residue no. 2096,
or hybridize to SEQ ID NO: 17 in the region from residue no. 130 to residue no. 1,
or hybridize to SEQ ID NO:18 in the region from residue no. 4834 to residue no. 4765,
or hybridize to SEQ ID NO:19 in the region from residue no. 349 to residue no. 220.

Furthermore, the present inventors identified a number of preferred primers to be used for PCR-reactions, preferably real-time-PCR-reactions, using the programme PRIMER on the sequences SEQ ID NO: 1-19. More specifically, SEQ ID NO: 39 and 40 are preferred forward and reverse primers, respectively, for amplification of SEQ ID NO: 1, resulting in a PCR*-product having a length of 206 nucleotides.
SEQ ID NO: 41 and 42 are preferred forward and reverse primers, respectively for amplification of SEQ ID NO: 2, resulting in a PCR-amplification-product of 201 nucleotides length,
SEQ ID NO: 43-44 are preferred forward and reverse primers, respectively for SEQ ID NO: 3, resulting in an amplification product of 203 nucleotides in length,
SEQ ID NO: 45-46, are preferred forward and reverse primers, respectively, for SEQ ID NO: 4, resulting in an amplification product of 208 nucleotides in length,
SEQ ID NO: 47 and 48 are preferred forward and reverse primers, respectively, for SEQ ID NO: 5, resulting in amplification product of 238 nucleotides in length,
SEQ ID NO: 49-50 are preferred forward and reverse primers, respectively, for SEQ ID NO: 6, resulting in an amplification product of 228 nucleotides in length,
SEQ ID NO: 51-52 are preferred forward and reverse primers, respectively, for SEQ ID NO: 3, resulting in an amplification product of 198 nucleotides in length,
SEQ ID NO: 53-54 are preferred forward and reverse primers, respectively, for SEQ ID NO: 8, resulting in an amplification product of 231 nucleotides in length,
SEQ ID NO: 55-56 are preferred forward and reverse primers for SEQ ID NO: 9, resulting, in an amplification product of 228 nucleotides in length,
SEQ ID NO: 57-58 are preferred forward and reverse primers for SEQ ID NO: 10, resulting, in an amplification product of 224 nucleotides in length,
SEQ ID NO: 59-60 are preferred forward and reverse primers, respectively, for SEQ ID NO: 11, resulting in an amplification product of 195 nucleotides in length,
SEQ ID NO: 61-62 are preferred forward and reverse primers for SEQ ID NO: 12, resulting, in an amplification product of 234 nucleotides in length,
SEQ ID NO: 63-64 are preferred forward and reverse primers for SEQ ID NO: 13, resulting, in an amplification product of 222 nucleotides in length,
SEQ ID NO: 65-66 are preferred forward and reverse primers for SEQ ID NO: 14, resulting, in an amplification product of 253 nucleotides in length,
SEQ ID NO: 67-68 are preferred forward and reverse primers, respectively, for SEQ ID NO: 15, resulting, in an amplification product of 191 nucleotides in length,
SEQ ID NO: 69-70 are preferred forward and reverse primers, respectively, for SEQ ID NO: 16, resulting, in an amplification product of 237 nucleotides in length,
SEQ ID NO: 71-72 are preferred forward and reverse primers, respectively, for SEQ ID NO: 17, resulting in an amplification product of 222 nucleotides in length,
SEQ ID NO: 73-74 are preferred forward and reverse primers, respectively, for SEQ ID NO: 18, resulting in an amplification product of 234 nucleotides in length, and
SEQ ID NO: 75-76 are preferred forward and reverse primers, respectively, for SEQ ID NO: 19, resulting in an amplification product of 219 nucleotides in length.

### Example 3

### Prototype gene-array

Customized RA specific MO cDNA or oligomer DNA based microarray includes selected RA-MO specific up and down regulated genes from SEQ ID 1 to SEQ ID 19 which are differentially expressed in RA vs. ND. The prototype(s) microarray(s) is/are designed so that 2 or better more than 8 replicates of each target probe are spotted or printed alongside with positive cDNA or oligomer target samples (housekeeping genes and commercial spike controls), negative controls, and guide dots. Housekeeping genes include 18S RNA (M10098), 28S RNA (U13369),Glycerol-aldehyde-3-phosphatase (M33197), beta-actin (X00351), gamma-actin (X04098), Glucose-6-phosphat dehydrogenase (NM_000402). A preferred protocol with typical hybridization conditions is as follows:

### Protocol for Microarray Hybridization of cDNA and Oligo Microarray Slides

### Solution Required:

- Pre-Fixing Buffer:
   - 10g BSA
   - 150 ml 20x SSC Solution
   - Fill to 1000 ml with DEPC treated H₂O
- 10 g BSA
   • 10g BSA
   • 150 ml 20x SSC Solution
   • Fill to 1000 ml with DEPC treated H₂O
- Solution 1:
   - 25 ml 20x SSC solution
   - 10 ml 10% SDS solution
   - Fill to 1000 ml with Milli-Q H₂O
- Solution 2:
   - 25 ml 20x SSC solution
   - 1000 µl 10% SDS solution
   - Fill to 1000 ml with Milli-Q water
- Solution 3:
   - 3 ml 20x SSC solution
   - Fill to 1000 ml with Milli-Q water.

### Experiment:

1. Use samples prepared according to Microarray labeling short. Doc
2. Turn on water bath to 55°C and one onto 42°C.
3. Pre-warm 45 ml of the pre-fixing buffer at 55°C for 20 mins.
4. Place the slide into a slide holder (Camlab) and place into the pre-fixing buffer. Turn the tube up-side down several times to remove air bubbles.
5. Incubate at 55°C for 1 hour.
6. Fill a 50 ml tube with Milli-Q highly filtered water.
7. Wash the slide in the water by lifting the slide holder up and down (2 times) in the tube containing the water.
8. Remove the slide from the holder, and place the slide into a rack.
9. Centrifuge the slide at 1000 rpm for 5 minutes to dry.
10. Place a Pyrex dish (or 2L beaker) into the microwave and bring water to near boiling, and then place the dish onto a hot plate, and bring to boil.
11. Turn off heat and immerse rack into basin containing boiling water, and leave for 2 minutes.
12. Remove rack, and place the rack into a glass chamber containing 95% Ethanol. Leave for 1 minute.
13. Centrifuge in rack at 1000 rpm for 5 minutes.
14. Fill a 50 ml tube with 45 ml of pre-hybridization buffer and place at 55°C to pre-warm for at least 20 minutes.
15. Add slide to a slide holder and place it into the tube containing pre-hybridization solution tube and incubate at 55°C for 25 minutes.
16. Fill two 50 ml tubes with 40 ml Milli-Q water and one with 100% Ethanol.
17. Remove slide and place into a tube containing Milli-Q water.
18. Raise and lower the slide holder 5 times to wash.
19. Repeat step 18 with second tube of water.
20. Place slide into tube containing 100% ethanol, and raise and lower the slide holder 5 times.
21. Remove and place in rack and centrifuge for 5 minutes at 1000 rpm.
22. Clean the hyb chamber (Array-IT) and remove dust by using compressed air.
23. Place the slide array side up in the hyb chamber.
24. Away from the array, clean a Lifter Slip coverslip (Erie Scientific) with compressed air to remove dust.
25. Place the coverslip onto the slide covering the array area making sure that the white strips are down creating a slight gap between the array and the coverslip.
26. Pipette 38 µl of sample prepared using Microarray Labeling. Doc at the front of the coverslip (half at each end, capillary action will take sample under).
27. Make sure no air bubbles are present and the whole array is covered.
28. Add 20 µl of3 x SSC into each end of a chamber once cleaned with Mill-Q water.
29. Close the hybridization chamber clamp lid down tightly (use screw driver if necessary).
30. Place whole apparatus in a water bath or oven at 42°C.
31. Leave ON (at least 10 hours).
32. Fill one 50 ml falcon tube with solution 1.
33. Add the slide to the tube, and shake off the cover slip by lifting the slide up and down.
34. Fill one glass box was filled with Solution 1, one with solution 2 and one with solution 3.
35. The rack was placed in the box containing solution 1.
36. Place the slide into the rack in the chamber filled with solution 1.
37. Cover the box with tin foil.
38. The whole box should then be placed on the shaker for 4 minutes mixing at a gentle speed.
39. Remove the rack and place in the box containing solution 2, cover the box with tin foil. Shake again for 4 minutes.
40. Remove the rack and place in the box containing solution 3, cover the box with tin foil. Shake again for 4 minutes.
41. Remove the rack, and centrifuge for 5 minutes at 1000 rpm (large Heraeus Megafuge).
42. Once dry place in dark box and take to scanner.

In exchange for a "standard" nucleic acid chip/microarray as described further above, a suspension array system may be used for the present invention. Such a suspension array system comprises beads on which capture nucleic acids have been immobilized. Hybridization events between a capture nucleic acid and a target nucleic acid can be detected e.g. using fluorimetry. One example of such a suspension array system is the Bio-Plex suspension array system.

The Bio-Plex suspension array system utilizes Luminex xMAP technology to permit the multiplexing of up to 100 different assays within a single sample. The system uses a liquid suspension array of 100 sets of 5.6 µm beads, each internally dyed with different ratios of two spectrally distinct fluorophores to assign it a unique spectral address. Each set of beads can be conjugated with a different capture molecule. The conjugated beads can then be mixed and incubated with sample in a microplate well to react with specific analytes. Capture molecules can include enzyme substrates, DNA, receptors, antigens, and antibodies. To detect and quantitate each captured analyte, a fluorescently labeled reporter molecule that specifically binds the analyte is added. Following incubation, the contents of each microplate well are drawn into the Bio-Plex array reader, and precision fluidics align the beads in single file through a flow cell, where two lasers excite the beads individually. One excites the dyes in each bead, identifying its spectral address, and the other excites the reporter molecule associated with the bead, which allows quantitation of the captured analyte. High-speed digital signal processors and Bio-Plex Manager software record the fluorescent signals simultaneously for each bead, translating the signals into data for each bead-based assay.

### Example 4

RT-PCR (reverse transcription-polymerase chain reaction) is the most sensitive technique for mRNA detection and quantitation currently available. Compared to the two other commonly used techniques for quantifying mRNA levels, Northern blot analysis and RNase protection assay, RT-PCR can be used to quantify mRNA levels from much smaller samples. In fact, this technique is sensitive enough to enable quantitation of RNA from a single cell and is used in several standard routine diagnostics.

In the present invention, real-time polymerase chain reaction with single or complex reaction may be used in order to detect and quantify levels of regulation of genes, which means that gene primers to the SEQ IDs 1 - 9 and SEQ IDs 10 - 19 e.g. some or all of the primers of SEQ IDNO: 39-76 will be used for monitoring differential gene expression and hence diagnosis of RA. Preferentially the length of amplified product is 140-260 nucleotides. For quantification of differential gene expression Thereby Taqman technology by primer quenching techniques or by incooperation of SYBR green marked nucleotides is preferred. TaqMan or molecular Beacon probes depend on the 5'- nuclease activity of the DNA polymerase used for PCR to hydrolyze an oligonucleotide that is hybridized to the target amplicon.

### SYBR Green for detecting and quantitating PCR-products in real-time reactions

SYBR Green provides the simplest and most economical format for detecting and quantitating PCR products in real-time reactions. SYBR Green binds double-stranded DNA, and upon excitation emits light. Thus, as a PCR product accumulates, fluorescence increases. The advantages of SYBR Green are that it is inexpensive, easy to use, and sensitive. The disadvantage is that SYBR Green will bind to any double-stranded DNA in the reaction, including primer-dimers and other non-specific reaction products, which results in an overestimation of the target concentration. For single PCR product reactions with well designed primers, SYBR Green can work extremely well, with spurious non-specific background only showing up in very late cycles.

SYBR Green is the most economical choice for real-time PCR product detection. Since the dye binds to double-stranded DNA, there is no need to design a probe for any particular target being analyzed. However, detection by SYBR Green requires extensive optimization. Since the dye cannot distinguish between specific and non-specific product accumulated during PCR, follow up assays are needed to validate results.

### TaqMan probes

TaqMan probes are an alternative means which may be used to detect and quantitate real-time PCR-products. TagMan oligonucleotides that have a fluorescent reporter dye attached to the 5' end and a quencher moeity coupled to the 3' end. These probes are designed to hybridize to an internal region of a PCR product. In the unhybridized state, the proximity of the fluor and the quench molecules prevents the detection of fluorescent signal from the probe. During PCR, when the polymerase replicates a template on which a TaqMan probe is bound, the 5'-nuclease activity of the polymerase cleaves the probe. This decouples the fluorescent and quenching dyes and Förster Resonance Energy Transfer (FRET) no longer occurs. Thus, fluorescence increases in each cycle, proportional to the amount of probe cleavage

### Molecular Beacons

Alternatively, for the detection and quantitation of real-time PCR products, Molecular Beacons may be used. Like TaqMan probes, Molecular Beacons also use FRET to detect and quantitate the synthesized PCR product via a fluor coupled to the 5' end and a quench attached to the 3' end of an oligonucleotide substrate. Unlike TaqMan probes, Molecular Beacons are designed to remain intact during the amplification reaction, and must rebind to target in every cycle for signal measurement. Molecular Beacons form a stem-loop structure when free in solution. Thus, the close proximity of the fluor and quench molecules prevents the probe from fluorescing. When a Molecular Beacon hybridizes to a target, the fluorescent dye and quencher are separated, FRET does not occur, and the fluorescent dye emits light upon irradiation.

Molecular Beacons, like TaqMan probes, can be used for multiplex assays by using spectrally separated fluor/quench moieties on each probe. As with TaqMan probes, Molecular Beacons can be expensive to synthesize, with a separate probe required for each target.

The features of the present invention disclosed in the specification, the claims and/or in the accompanying drawings, may, both separately, and in any combination thereof, be material for realising the invention in various forms thereof.

## Claims

1. A composition comprising at least the following nucleic acid sequences: 213649_at (splicing factor, arginine/serine-rich 7; SEQ ID NO:1), 219607_s_at (membrane-spanning 4-domains, subfamily A, member 4; SEQ ID NO:2), 212179_at (chromosome 6 open reading frame; SEQ ID NO:3), 205239_at (amphiregulin (schwannoma-derived growth factor)) SEQ ID NO:4), 205950_s_at (carbonic anhydrase I) SEQ ID NO:5), 208911_s_at (pyruvate dehydrogenase E-1 beta subunit; SEQ ID NO:6), 211560_s_at (aminolevulinate, delta-, synthase 2; SEQ ID NO:7), 202464_s_at (6-phosphofructo-2-kinase/fructose-2,6-bisphosphatase 3; SEQ ID NO:8), and 212706_at (RAS p21 protein activator 4; SEQ ID NO:9), and/or the complementary counterparts thereof, and/or partial sequences thereof comprising 18 contiguous nucleotides or more and/or the complementary counterparts thereof.

2. Composition according to claim 1, wherein said partial sequences of SEQ ID NO: 1-9 are selected from the group comprising SEQ ID NO:20-28 and oligomers of up to 70 nucleotides
which hybridize to SEQ ID NO:1 in the region from residue no. 131 to residue no. 1,
or hybridize to SEQ ID NO:2 in the region from residue no. 255 to residue no. 33,
or hybridize to SEQ ID NO:3 in the region from residue no. 409 to residue no. 280,
or hybridize to SEQ ID NO:4 in the region from residue no. 310 to residue no. 181,
or hybridize to SEQ ID NO:5 in the region from residue no. 130 to residue no. 1,
or hybridize to SEQ ID NO:6 in the region from residue no. 146 to residue no. 17,
or hybridize to SEQ ID NO:7 in the region from residue no. 1463 to residue no. 1334,
or hybridize to SEQ ID NO:8 from residue no. 136 to residue no. 7,
or hybridize to SEQ ID NO:9 from residue no. 1490 to residue no. 1361.

3. Composition according to any of claims 1-2, furthermore comprising one or more of the following additional sequences: 205987_at (CD1C antigen, c polypeptide; SEQ ID NO:10), 214280_x_at (heterogeneous nuclear ribonucleoprotein A1, SEQ ID NO:11), 218204_s_at (FYVE and coiled-coil domain; SEQ ID NO:12), and/or the complementary counterparts thereof, and/or partial sequences thereof comprising 18 contiguous nucleotides or more and/or complementary counterparts thereof.

4. Composition according to claim 3 wherein said partial sequences of SEQ ID NO:10-12 are selected from the group comprising SEQ ID NO:29-31 and oligomers of up to 70 nucleotides
which hybridize to SEQ ID NO: 10 in the region from residue no. 769 to residue no. 640,
or hybridize to SEQ ID NO: 11 in the region from residue no. 130 to residue no. 1,
or hybridize to SEQ ID NO: 12 in the region from residue no. 130 to residue no. 1.

5. Composition according to any of the foregoing claims, furthermore comprising one or more of the following nucleic acid sequences:
207332_s_at (transferrin receptor (p90, CD71); SEQ ID NO:13), 203115_at (ferrochelatase; SEQ ID NO:14), 219093_at (hypothetical protein FLJ20701; SEQ ID NO:15), 219714_s_at (calcium channel, (voltage-dependent); SEQ ID NO:16), 221622_s_at (uncharacterized hypothalamus protein HT007; SEQ ID NO:17), 221748_s_at (tensin; SEQ ID NO: 18), and 201890_at (ribonucleotide reductase M2 polypeptide; SEQ ID NO: 19), and/or the complementary counterparts thereof, and/or partial sequences thereof comprising 18 nucleotides or more and/or complementary counterparts.

6. Composition according to claim 5, wherein said partial sequences of SEQ ID NO:13-19 are selected from the group comprising SEQ ID NO:32-38 and oligomers of up to 70 nucleotides
which hybridize to SEQ ID NO: 13 in the region from residue no. 192 to residue no. 63,
or hybridize to SEQ ID NO:14 in the region from residue no. 204 to residue no. 75,
or hybridize to SEQ ID NO:15 in the region from residue no. 226 to residue no. 97,
or hybridize to SEQ ID NO: 16 in the region from residue no. 2225 to residue no. 2096,
or hybridize to SEQ ID NO: 17 in the region from residue no. 130 to residue no. 1,
or hybridize to SEQ ID NO:18 in the region from residue no. 4834 to residue no. 4765,
or hybridize to SEQ ID NO: 19 in the region from residue no. 349 to residue no. 220.

7. Composition according to claim 1, wherein said complementary counterparts of said partial sequences comprising 18 contiguous nucleotides are 9 sets of primers, each set of primers comprising a forward primer and a reverse primer, each set of primers being able to hybridize to one nucleic acid sequence selected from SEQ ID NO: 1-9, such that different sets of primers are able to hybridize to different nucleic acid sequences and all 9 sets of primers in their entirety are able to hybridize to SEQ ID NO: 1-9, said composition further being suitable to be used in a nucleic acid amplification reaction, preferably a polymerase chain reaction, said primers being selected such that the resulting amplification reaction product(s) are 140-260 nucleotides in length.

8. Composition according to claim 7, wherein said 9 sets of primers are SEQ ID NO:39-56.

9. Composition according to any of claims 7-8, furthermore comprising one to three additional sets of primers, each additional set of primers comprising a forward primer and a reverse primer, each additional set of primers being able to hybridize to one nucleic acid sequence selected from SEQ ID NO:10-12, such that different sets of primers are able to hybridize to different nucleic acid sequences, said composition further being suitable to be used in a nucleic acid amplification reaction, preferably a polymerase chain reaction, said primers being selected such that the resulting amplification reaction product(s) are 140-260 nucleotides in length.

10. Composition according to claim 9, wherein said one to three additional sets of primers are selected from the group comprising SEQ ID NO:57-62.

11. Composition according to any of claims 7-10, furthermore comprising one to seven additional sets of primers, each additional set of primers comprising a forward primer and a reverse primer, each additional set of primers being able to hybridize to one nucleic acid sequence selected from SEQ ID NO: 13-19, such that different sets of primers are able to hybridize to different nucleic acid sequences , said composition further being suitable to be used in a nucleic acid amplification reaction, preferably a polymerase chain reaction, said primers being selected such that the resulting amplification reaction product(s) are 140-260 nucleotides in length.

12. Composition according to claim 11, wherein said one to seven additional sets of primers are selected from the group comprising SEQ ID NO: 63-76.

13. Composition according to any of claims 7-12, further comprising reagents for nucleic acid amplification and a detectable nucleic acid binding agent.

14. A solid matrix having a surface on which a nucleic acid composition as defined in any of claims 1-6 or a nucleic acid composition as defined in any of claims 7-13 has been immobilized.

15. Matrix according to claim 14, which is a substrate having a surface, preferably a planar substrate.

16. Matrix according to claim 14, which is a set of beads suitable for use in a high-throughput assay, said beads having surfaces on which said nucleic acid composition as defined in any of claims 1-6 and/or in any of claims 7-13 has been immobilized.

17. Use of the composition according to any of claims 1-13 and/or of the matrix according to any of claims 14-16 for in-vitro-diagnosis, prognosis and/or detection of an inflammatory process, in particular an inflammatory disease, in a patient.

18. Use according to claim 17, wherein said inflammatory process or disease is rheumatoid arthritis (RA).

19. Use according to claim 17, wherein said inflammatory process or disease is a chronic inflammatory disease, other than rheumatoid arthritis, involving monocytes/macrophages.

20. Use according to any of claims 17-19 for the monitoring of monocyte/macrophage activation in a body fluid or body tissue of an organism.


SEQUENCE LISTING

<110> Oligene GmbH
      Stuhlmüller, Bruno

<120> A composition of nucleic acid sequences, specific for inflammatory
      disease, in particular rheumatoid arthritis.

<130> FB16443

<160> 76

<170> PatentIn version 3.2

<210> 1
<211> 2754
<212> DNA
<213> Homo sapiens

<400> 1
actcgagccc tgggcgctgc ttgctaaaga gccgagcacg cgggtctgtc atcatgtcgc      60

gttacgggcg gtacggagga gaaaccaagg tgtatgttgg taacctggga actggcgctg     120

gcaaaggaga gttagaaagg gctttcagtt attatggtcc tttaagaact gtatggattg     180

cgagaaatcc tccaagattt gcctttgtgg aattcgaaga tcctagagat gcagaagatg     240

cagtacgagg actggatgga aaggtgattt gtggctcccg agtgagggtt gaactatcga     300

caggcatgcc tcggagatca cgttttgata gaccacctgc ccgacgtccc tttgatccaa     360

atgatagatg ctatgagtgt ggcgaaaagg gacattatgc ttatgattgt catcgttaca     420

gccggcgaag aagaagcagg tttcttcgtt tgagtcagtc gccttgattc agaatgtcac     480

gagccttatg atatcatgct gaggcgcctt gcaaatccga caattaagat cctcctagac     540

cttgaggtga tcagcataag aggccagatc ccctcgagtc atctacacct agcttcacct     600

tattctttaa agggcagaaa atttgagacg gtgatcgccg taacagtaaa tttggcttac     660

aattggggcc cccctccggt ttagaaagag gaacaccaga ttgaccacat tcccaactag     720

aaaaatcttc ttgcgtcaat caagcctcac ctggctcatt tggctgtcag tttgatcgtc     780

gttagattga agaaaacatc tagatgcagc gatcggctat agatacttct agatcgtcta     840

gatctactag accatgggcc aaagagggtc gacctgcaaa cttgcaaggt cacggtctag     900

atcacattct cgatccagag gaaggcgata ctctcgctca cgcagcagga gcaggggacg     960

aaggtcaagg tcagcatctc ctcgacgatt aagatctatc tctcttcgta gatcaagatc    1020

agcttcactc agaagatcta ggtctggttc tataaaagga tcgaggtatt tccaatcccc    1080

gtcgaggtca agatcaagat ccaggtctat ttcacgacca agaagcagcc gatcaaagtc    1140

cagatctcca tctccaaaaa gaagtcgttc cccatcagga agtcctcgca gaagtgcaag    1200

tcctgaaaga atggactgaa gctctcaagt tcacccttta gggaaaagtt attttgttta    1260

cattattata agggatttgt gatgtctgta aagtgtaacc taggaaagat aattcaacca    1320

tctaatcaaa atggatctgg attactatgt aaattcacag cagtaagata atataaattt 1380

tgttgaatgt attaacatca tatggtctga aaatgtgggt ttttatttgg cacatttaaa 1440

taaaatgttt ctaactagat ttttgatttg tgttcaatat taacacttct taatttgata 1500

tatttgagag tcagacatta taattgttaa ccttattcat acatacctac attcagaatt 1560

gaaaggtgtt ggttaagtct tgaacatcac tattctatgc ataaaacttg gccaggatct 1620

taagggactt tgaaaattcc atcttaccct tgtagctctg ggtaagatga cctgagtccc 1680

ttatgataca gcctgaatgc atcatgacag atccttaagt tagctaatcc gtttgaagtt 1740

ggtgttagta ggtattgtat gatcagtggt gaagcaagta ggaccactga tgtgtctaaa 1800

tgagcatgac aggaactaaa cgaaactgat taaatgtatg agaaatagaa actgatttct 1860

ggatgatctt tatactaatt gcagctttca ggctactagg tggcatagtg ttaattagga 1920

ctccccaaga tatgggagt tctactctca atggtcttgt ttctttgctt tctacattag 1980

ttaaccagtt ttataccaaa aaatgcatgt ttgaggaatt gtctgaaatt gggacaaaac 2040

accttcatgt aaaccagctt tgcaaaattt tccagcccag atactcttca tctattcaaa 2100

tggattgtct tattctgagc aaagacctgt tgttaatctt caagctaggt tttgcagttc 2160

ccaaccacaa cattcttcta ttttgccagg ctggtgcaaa gtaattaaag atgtcaatca 2220

gaaatgtcaa tgagactaaa gtggttttgt aaatctcagc tatatttagc aacactccat 2280

gtagctaata ttttttggta gcatctggta gaccttagaa tgttacatag ccagtaggtt 2340

ctttattcaa attttaagta tcttaagaat agtagggcag taacagttac ttttgagagt 2400

tttctggtca agcttttacc aggcattctc tagccttggt acaaaaaaaa aaaaaacctg 2460

ctggttgcgc agatacctag gcttgtccat tttatgcatt tcagcaaagt cattggatac 2520

tattgcaact tgggaatact ggtctgcatc aagtttattc ggtagtttga ccgctagtat 2580

gttggaagtt atttggattg tttttggaat tttgactggc tgaattatgg ttggtataaa 2640

gttatgtgta taactggcag gcttatttat ctgttgcact tggttagctt taattgttct 2700

gtattattta aagataagtt tactcaacaa taaatctgca gagattgaac aaat 2754

<210> 2
<211> 1619
<212> DNA
<213> Homo sapiens

<400> 2
aacattcctg caaatggttt caatatatgc agatgtctcg atataggaat gaaattacgt 60

ctttggaaca acttaaataa gtcaaatata cttggagctt taaaaattaa aaggagagag 120

attcgagcac cttttctgct gccatgacaa ccatgcaagg aatggaacag gccatgccag 180

gggctggccc tggtgtgccc cagctgggaa acatggctgt catacattca catctgtgga 240

aaggattgca agagaagttc ttgaagggag aacccaaagt ccttggggtt gtgcagattc 300

tgactgccct gatgagcctt agcatgggaa taacaatgat gtgtatggca tctaatactt 360

atggaagtaa ccctatttcc gtgtatatcg ggtacacaat ttgggggtca gtaatgttta 420

ttatttcagg atccttgtca attgcagcag gaattagaac tacaaaaggc ctggtccgag 480

gtagtctagg aatgaatatc accagctctg tactggctgc atcagggatc ttaatcaaca 540

catttagctt ggcgttttat tcattccatc acccttactg taactactat ggcaactcaa 600

ataattgtca tgggactatg tccatcttaa tgggtctgga tggcatggtg ctcctcttaa 660

gtgtgctgga attctgcatt gctgtgtccc tctctgcctt tggatgtaaa gtgctctgtt 720

gtacccctgg tggggttgtg ttaattctgc catcacattc tcacatggca gaaacagcat 780

ctcccacacc acttaatgag gtttgaggcc accaaaagat caacagacaa atgctccaga 840

aatctatgct gactgtgaca caagagcctc acatgagaaa ttaccagtat ccaacttcga 900

tactgataga cttgttgata ttattattat atgtaatcca attatgaact gtgtgtgtat 960

agagagataa taaattcaaa attatgttct catttttttc cctggaactc aataactcat 1020

ttcactggct ctttatcgag agtactagaa gttaaattaa taaataatgc atttaatgag 1080

gcaacagcac ttgaaagttt ttcattcatc ataagaactt tatataaagg cattacattg 1140

gcaaataagg tttggaagca gaagagcaaa aaaaagatat tgttaaaatg aggcctccat 1200

gcaaaacaca tacttccctc ccatttattt aacttttttt ttctcctacc tatggggacc 1260

aaagtgcttt ttccttcagg aagtggagat gcatggccat ctccccctcc ctttttcctt 1320

ctcctgcttt tctttcccca tagaaagtac cttgaagtag cacagtccgt ccttgcatgt 1380

gcacgagcta tcatttgagt aaaagtatac atggagtaaa aatcatatta agcatcagat 1440

tcaacttata ttttctattt catcttcttc ctttcccttc tcccaccttc tactgggcat 1500

aattatatct taatcatata tggaaatgtg caacatatgg tatttgttaa atacgtttgt 1560

ttttattgca gagcaaaaat aaatcaaatt agaagcaata aaaaaaaaaa aaaaaaaaa 1619

<210> 3
<211> 2663
<212> DNA
<213> Homo sapiens

<400> 3
gatagaaaat caagtatatt ttaaaagaag ggatgtggga tcaaggagga cagccttggc 60

agcagtggcc cttgaaccag caacaatgga tgcagtcatt ccagcaccaa caggatccaa 120

gccagattga ttgggctgca ttggcccaag cttggattgc ccaaagagaa gcttcaggac 180

agcaaagcat ggtagaacaa ccaccaggaa tgatgccaaa tggacaagat atgtctacaa 240

tggaatctgg tccaaacaat catgggaatt tccaagggga ttcaaacttc aacagaatgt 300

ggcaaccaga atggggaatg catcagcaac ccccacaccc ccctccagat cagccatgga 360

tgccaccaac accaggccca atggacattg ttcctccttc tgaagacagc aacagtcagg 420

acagtgggga atttgcccct gacaacaggc atatatttaa ccagaacaat cacaactttg 480

gtggaccacc cgataatttt gcagtggggc cagtgaacca gtttgactat cagcatgggg 540

ctgcttttgg tccaccgcaa ggtggatttc atcctcctta ttggcaacca ggacctccag 600

gacctccagc acctccccag aatcgaagag aaaggccatc atcattcagg gatcgtcagc 660

gttcacctat tgcacttcct gtgaagcagg agcctccaca aattgacgca gtaaaacgca 720

ggactcttcc cgcttggatt cgcgaaggtc ttgaaaaaat ggaacgtgaa aagcagaaga 780

aattggagaa agaaagaatg gaacaacaac gttcacaatt gtccaaaaaa gaaaaaaagg 840

ccacagaaga tgctgaagga ggggatggcc ctcgtttacc tcagagaagt aaatttgata 900

gtgatgagga agaagaagac actgaaaatg ttgaggctgc aagtagtggg aaagtcacca 960

gaagtccatc cccagttcct caagaagagc acagtgaccc tgagatgact gaagaggaga 1020

aagagtatca aatgatgttg ctgacaaaaa tgcttctaac agaaattctg ctggatgtca 1080

cagatgaaga aatttattac gtagccaaag atgcacaccg caaagcaacg aaagctcctg 1140

caaaacagct ggcacagtcc agtgcactgg cttccctcac tggactcggt ggactgggtg 1200

gttatggatc aggagacagt gaagatgaga ggagtgacag aggatctgag tcatctgaca 1260

ctgatgatga agaattacgg catcgaatcc ggcaaaaaca ggaagctttt tggagaaaag 1320

aaaaagaaca gcagctatta catgataaac agatggaaga agaaaagcag caaacagaaa 1380

gggttacaaa agagatgaat gaatttatcc ataaagagca aaatagttta tcactactag 1440

aagcaagaga agcagacggt gatgtggtta atgaaaagaa gagaactcca aatgaaacca 1500

catcagtttt agaaccaaaa aaagagcata aagaaaaaga aaaacaagga aggagtaggt 1560

cgggaagttc tagtagtggt agtcccagta gcaatagcag aactagtagt actagtagta 1620

ctgtctctag ctcttcatac agttctagct caggtagtag tcgtacttct tctcggtctt 1680

cttctcctaa aaggaaaaag agacacagta ggagtagatc tccaacaatc aaagctagac 1740

gtagcaggag tagaagctat tctcgcagaa ttaaaataga gagcaatagg gctagggtaa 1800

agattagaga tagaaggaga tctaatagaa atagcattga aagagaaaga cgacgaaatc 1860

ggagtccttc ccgagagaga cgtagaagta gaagtcgctc aagggataga cgaaccaatc 1920

gtgccagtcg cagtaggagt cgagataggc gtaaaattga tgatcaacgt ggaaatctta 1980

gtgggaacag tcataagcat aaaggtgagg ctaaagaaca agagaggaaa aaggagagga 2040

gtcgaagtat agataaagat aggaaaaaga aagacaaaga aagggaacgt gaacaggata 2100

aaagaaaaga gaaacaaaaa agggaagaaa aagattttaa gttcagtagt caggatgata 2160

gattaaaaag gaaacgagaa agtgaaagaa cattttctag gagtggttct atatctgtta 2220

aaatcataag acatgattct agacaggata gtaagaaaag tactaccaaa gatagtaaaa 2280

aacattcagg ctctgattct agtggaagga gcagttctga gtctccagga agtagcaaag 2340

aaaagaaggc taagaagcct aaacatagtc gatcgcgatc cgcggagaaa tctcaaaggt 2400

ctggtaagaa ggcaagccgc aaacacaagt ctaagtcccg atcaaggtag tatacttttt 2460

aaagtatttt gtctgatttt taaaaaaaat tgactgaatt tattcaaagt tgaaagtgtc 2520

ctttctctct ctctttaata aactcagttt ggtacttgat aaataatcat agtcttaaat 2580

gttagaaatc ctatataata ttatttattt aaaattgcag atttttaatt taaaatacat 2640

ttttattttt aaatttgtc ttt 2663

<210> 4
<211> 1270
<212> DNA
<213> Homo sapiens

<400> 4
agacgttcgc acacctgggt gccagcgccc cagaggtccc gggacagccc gaggcgccgc 60

gcccgccgcc ccgagctccc caagccttcg agagcggcgc acactcccgg tctccactcg 120

ctcttccaac acccgctcgt tttggcggca gctcgtgtcc cagagaccga gttgccccag 180

agaccgagac gccgccgctg cgaaggacca atgagagccc cgctgctacc gccggcgccg 240

gtggtgctgt cgctcttgat actcggctca ggccattatg ctgctggatt ggacctcaat 300

gacacctact ctgggaagcg tgaaccattt tctggggacc acagtgctga tggatttgag 360

gttacctcaa gaagtgagat gtcttcaggg agtgagattt cccctgtgag tgaaatgcct 420

tctagtagtg aaccgtcctc gggagccgac tatgactact cagaagagta tgataacgaa 480

ccacaaatac ctggctatat tgtcgatgat tcagtcagag ttgaacaggt agttaagccc 540

ccccaaaaca agacggaaag tgaaaatact tcagataaac ccaaaagaaa gaaaaaggga 600

ggcaaaaatg gaaaaaatag aagaaacaga aagaagaaaa atccatgtaa tgcagaattt 660

caaaatttct gcattcacgg agaatgcaaa tatatagagc acctggaagc agtaacatgc 720

aaatgtcagc aagaatattt cggtgaacgg tgtggggaaa agtccatgaa aactcacagc 780

atgattgaca gtagtttatc aaaaattgca ttagcagcca tagctgcctt tatgtctgct 840

gtgatcctca cagctgttgc tgttattaca gtccagctta gaagacaata cgtcaggaaa 900

tatgaaggag aagctgagga acgaaagaaa cttcgacaag agaatggaaa tgtacatgct 960

atagcataac tgaagataaa attacaggat atcacattgg agtcactgcc aagtcatagc 1020

cataaatgat gagtcggtcc tctttccagt ggatcataag acaatggacc ctttttgtta 1080

tgatggtttt aaactttcaa ttgtcacttt ttatgctatt tctgtatata aaggtgcacg 1140

aaggtaaaaa gtatttttc aagttgtaaa taatttattt aatatttaat ggaagtgtat 1200

ttatttaca gctcattaaa cttttttaac caaacagaaa aaaaaaaaa aaaaaaaaa 1260

aaaaaaaaa 1270

<210> 5
<211> 1264
<212> DNA
<213> Homo sapiens

<400> 5
gtggtaccca gtcctcaggt gcaaccccct gcgtggtcct ctgtggcagc cttctctcat 60

tcagagctgt tttccacaga ggtagtgaaa agaactggat tttcaagttc actttgcaag 120

agaaaaagaa aactcagtag aagataatgg caagtccaga ctggggatat gatgacaaaa 180

atggtcctga acaatggagc aagctgtatc ccattgccaa tggaaataac caatcccctg 240

ttgatattaa aaccagtgaa accaaacatg acacctctct gaaacctatt agtgtctcct 300

acaacccagc cacagccaaa gaaattatca atgtggggca ttctttccat gtaaattttg 360

aggacaacga taaccgatca gtgctgaaag gtggtccttt ctctgacagc tacaggctct 420

ttcagtttca ttttcactgg ggcagtacaa atgagcatgg ttcagaacat acagtggatg 480

gagtcaaata ttctgccgag cttcacgtag ctcactggaa ttctgcaaag tactccagcc 540

ttgctgaagc tgcctcaaag gctgatggtt tggcagttat tggtgttttg atgaaggttg 600

gtgaggccaa cccaaagctg cagaaagtac ttgatgccct ccaagcaatt aaaaccaagg 660

gcaaacgagc cccattcaca aattttgacc cctctactct ccttccttca tccctggatt 720

tctggaccta ccctggctct ctgactcatc ctcctcttta tgagagtgta acttggatca 780

tctgtaagga gagcatcagt gtcagctcag agcagctggc acaattccgc agccttctat 840

caaatgttga aggtgataac gctgtcccca tgcagcacaa caaccgccca acccaacctc 900

tgaagggcag aacagtgaga gcttcatttt gatgattctg agaagaaact tgtccttcct 960

caagaacaca gccctgcttc tgacataatc cagttaaaat aataattttt aagaaataaa 1020

tttatttcaa tattagcaag acagcatgcc ttcaaatcaa tctgtaaaac taagaaactt 1080

aaattttagt tcttactgct taattcaaat aataattagt aagctagcaa atagtaatct 1140

gtaagcataa gcttatctta aattcaagtt tagtttgagg aattctttaa aattacaact 1200

aagtgatttg tatgtctatt tttttcagtt tatttgaacc aataaaataa ttttatctct 1260

ttct 1264

<210> 6
<211> 1501
<212> DNA
<213> Homo sapiens

<400>  6
atagaggaca cgaccaagat ggcggcggtg tctggcttgg tgcggagacc ccttcgggag      60

gtctccgggc tgctgaagag gcgctttcac tggaccgcgc cggctgcgct gcaggtgaca     120

gttcgtgatg ctataaatca gggtatggat gaggagctgg aaagagatga gaaggtattt     180

ctgcttggag aagaagttgc ccagtatgat ggggcataca aggttagtcg agggctgtgg     240

aagaaatatg gagacaagag gattattgac actcccatat cagagatggg ctttgctgga     300

attgctgtag gtgcagctat ggctgggttg cggcccattt gtgaatttat gaccttcaat     360

ttctccatgc aagccattga ccaggttata aactcagctg ccaagaccta ctacatgtct     420

ggtggccttc agcctgtgcc tatagtcttc aggggaccca atggtgcctc agcaggtgta     480

gctgcccagc actcacagtg ctttgctgcc tggtatgggc actgcccagg cttaaaggtg     540

gtcagtccct ggaattcaga ggatgctaaa ggacttatta aatcagccat tcgggataac     600

aatccagtgg tggtgctaga gaatgaattg atgtatgggg ttcctttga atttctcccg     660

gaagctcagt caaaagattt tctgattcct attggaaaag ccaaaataga aaggcaagga     720

acacatataa ctgtggtttc ccattcaaga cctgtgggcc actgcttaga agctgcagca     780

gtgctatcta aagaaggagt tgaatgtgag gtgataaata tgcgtaccat tagaccaatg     840

gacatggaaa ccatagaagc cagtgtcatg aagacaaatc atcttgtaac tgtggaagga     900

ggctggccac agtttggagt aggagctgaa atctgtgcca ggatcatgga aggtcctgcg     960

ttcaatttcc tggatgctcc tgctgttcgt gtcactggtg ctgatgtccc tatgccttat    1020

gcaaagattc tagaggacaa ctctatacct caggtcaaag acatcatatt tgcaataaag    1080

aaaacattaa atatttagtt tggacttgaa tatcaagtcg ttgaaattta tttgaaatac    1140

ttgctggcac tgcacctgga tttgtactgc aagacctgac tattcataaa ggaaaacgat    1200

ttctaaagca acagcaggta tttttgtaca gggaagttta aatgtgtttg tgtatggaaa    1260

actctccact ctcctcccct agatgccatg cttccttttg tctgttacgg ttgccatgtt    1320

ctttgaataa caaattatat cacattttat cctctctcac cacaaggaca aagtatggat    1380

gtggcagagt cctgatgaaa gatgtatcca aacaagataa cttatatgta taaaattaaa    1440

gcatataata cacatttact gttagtttgt tttgataagg aataaaggaa tttctaacat    1500

g                                                                    1501

<210>  7
<211>  1937
<212>  DNA
<213>  Homo sapiens

<400>  7
cacctgtcat tcgttcgtcc tcagtgcagg gcaacaggac tttaggttca agatggtgac      60

tgcagccatg ctgctacagt gctgcccagt gcttgcccgg ggccccacaa gcctcctagg     120

caaggtggtt aagactcacc agttcctgtt tggtattgga cgctgtccca tcctggctac 180

ccaaggacca aactgttctc aaatccacct taaggcaaca aaggctggag gagattctcc 240

atcttgggcg aagggccact gtcccttcat gctgtcggaa ctccaggatg ggaagagcaa 300

gattgtgcag aaggcagccc cagaagtcca ggaagatgtg aaggctttca agacagatct 360

gcctagctcc ctggtctcag tcagcctaag gaagccattt tccggtcccc aggagcagga 420

gcagatctct gggaaggtca cacacctgat tcagaacaat atgcctggaa actatgtctt 480

cagttatgac cagtttttca gggacaagat catggagaag aaacaggatc acacctaccg 540

tgtgttcaag actgtgaacc gctgggctga tgcatatccc tttgcccaac atttctttga 600

ggcatctgtg gcctcaaagg atgtgtccgt ctggtgtagt aatgattacc tgggcatgag 660

ccgacaccct caggtcttgc aagccacaca ggagaccctg cagcgtcatg gtgctggagc 720

tggtggcacc cgcaacatct caggcaccag taagtttcat gtggagcttg agcaggagct 780

ggctgagctg caccagaagg actcagccct gctcttctcc tcctgctttg ttgccaatga 840

ctctactctc ttcaccttgg ccaagatcct gccagggtgc gagatttact cagacgcagg 900

caaccatgct tccatgatcc aaggtatccg taacagtgga gcagccaagt ttgtcttcag 960

gcacaatgac cctgaccacc taaagaaact tctagagaag tctaacccta agatacccaa 1020

aattgtggcc tttgagactg tccactccat ggatggtgcc atctgtcccc tcgaggagtt 1080

gtgtgatgtg tcccaccagt atggggccct gaccttcgtg gatgaggtcc atgctgtagg 1140

actgtatggg tcccggggcg ctgggattgg ggagcgtgat ggaattatgc ataagattga 1200

catcatctct ggaactcttg gcaaggcctt tggctgtgtg ggcggctaca ttgccagcac 1260

ccgtgacttg gtggacatgg tgcgctccta tgctgcaggc ttcatcttta ccacttctct 1320

gccccccatg gtgctctctg gagctctaga atctgtgcgg ctgctcaagg gagaggaggg 1380

ccaagccctg aggcgagccc accagcgcaa tgtcaagcac atgcgccagc tactcatgga 1440

caggggcctt cctgtcatcc cctgccccag ccacatcatc cccatccggg tgggcaatgc 1500

agcactcaac agcaagctct gtgatctcct gctctccaag catggcatct atgtgcaggc 1560

catcaactac ccaactgtcc cccggggtga agagctcctg cgcttggcac cctcccccca 1620

ccacagccct cagatgatgg aagattttgt ggagaagctg ctgctggctt ggactgcggt 1680

ggggctgccc ctccaggatg tgtctgtggc tgcctgcaat ttctgtcgcc gtcctgtaca 1740

ctttgagctc atgagtgagt gggaacgttc ctacttcggg aacatggggc cccagtatgt 1800

caccacctat gcctgagaag ccagctgcct aggattcaca ccccacctgc gcttcacttg 1860

ggtccaggcc tactcctgtc ttctgctttg ttgtgtgcct ctagctgaat tgagcctaaa 1920

aataaagcac aaaccac 1937

<210> 8
<211> 4497
<212> DNA
<213> Homo sapiens

<400> 8
agccaagccg gagaggaggc gagcggcagg gcctggtggc gagagcgcgg ctgtcactgc 60

gcccgagcat cccagagctt tccgagcgga cgagccggcc gtgccgggca tccccagcct 120

cgctaccctc gcagcacacg tcgagccccg cacaggcgag ggtccggaac ttagcccaaa 180

gcacgtttcc cctggcagcg caggaaacgc ccggccgcgc gccggcgcac gcccccctct 240

cctcctttgt tccgggggtc ggcggccgct ctcctgccag cgtcgggatc tcggccccgg 300

gaggcgggcc gtcgggcgca gccgcgaaga tgccgttgga actgacgcag agccgagtgc 360

agaagatctg ggtgcccgtg gaccacaggc cctcgttgcc cagatcctgt gggccaaagc 420

tgaccaactc ccccaccgtc atcgtcatgg tgggcctccc cgcccggggc aagacctaca 480

tctccaagaa gctgactcgc tacctcaact ggattggcgt ccccacaaaa gtgttcaacg 540

tcggggagta tcgccgggag gctgtgaagc agtacagctc ctacaacttc ttccgccccg 600

acaatgagga agccatgaaa gtccggaagc aatgtgcctt agctgccttg agagatgtca 660

aaagctacct ggcgaaagaa gggggacaaa ttgcggtttt cgatgccacc aatactacta 720

gagagaggag acacatgatc cttcattttg ccaaagaaaa tgactttaag gcgtttttca 780

tcgagtcggt gtgcgacgac cctacagttg tggcctccaa tatcatggaa gttaaaatct 840

ccagcccgga ttacaaagac tgcaactcgg cagaagccat ggacgacttc atgaagagga 900

tcagttgcta tgaagccagc taccagcccc tcgaccccga caaatgcgac agggacttgt 960

cgctgatcaa ggtgattgac gtgggccgga ggttcctggt gaaccgggtg caggaccaca 1020

tccagagccg catcgtgtac tacctgatga acatccacgt gcagccgcgt accatctacc 1080

tgtgccggca cggcgagaac gagcacaacc tccagggccg catcgggggc gactcaggcc 1140

tgtccagccg gggcaagaag tttgccagtg ctctgagcaa gttcgtggag gagcagaacc 1200

tgaaggacct gcgcgtgtgg accagccagc tgaagagcac catccagacg gccgaggcgc 1260

tgcggctgcc ctacgagcag tggaaggcgc tcaatgagat cgacgcgggc gtctgtgagg 1320

agctgaccta cgaggagatc agggacacct accctgagga gtatgcgctg cgggagcagg 1380

acaagtacta ttaccgctac cccaccgggg agtcctacca ggacctggtc cagcgcttgg 1440

agccagtgat catggagctg gagcggcagg agaatgtgct ggtcatctgc caccaggccg 1500

tcctgcgctg cctgcttgcc tacttcctgg ataagagtgc agaggagatg ccctacctga 1560

aatgccctct tcacaccgtc ctgaaactga cgcctgtcgc ttatggctgc cgtgtggaat 1620

ccatctacct gaacgtggag tccgtctgca cacaccggga gaggtcagag gatgcaaaga 1680

agggacctaa cccgctcatg agacgcaata gtgtcacccc gctagccagc cccgaaccca 1740
ccaaaaagcc tcgcatcaac agctttgagg agcatgtggc ctccacctcg gccgccctgc 1800
ccagctgcct gcccccggag gtgcccacgc agctgcctgg acaaaacatg aaaggctccc 1860
ggagcagcgc tgactcctcc aggaaacact gaggcagacg tgtcggttcc attccatttc 1920
catttctgca gcttagcttg tgtcctgccc tccgcccgag gcaaaacgta tcctgaggac 1980
ttcttccgga gagggtgggg tggagcagcg gggagcctt ggccgaagag aaccatgctt 2040
ggcaccgtct gtgtcccctc ggccgctgga caccagaaag ccacgtgggt ccctggcgcc 2100
ctgcctttag ccgtggggcc cccacctcca ccctctgggt ttcctaggaa tgtccagcct 2160
cggagacctt cacaaagcct tgggagggtg atgagtgctg gtcctgacaa gaggccgctg 2220
gggacactgt gctgttttgt ttcgtttctg tgatctcccg gcacgtttgg agctgggaag 2280
accacactgg tggcagaatc ctaaaattaa aggaggcagg ctcctagttg ctgaaagtta 2340
aggaatgtgt aaaacctcca cgtgactgtt tggtgcatct tgacctggga agacgcctca 2400
tgggaacgaa cttggacagg tgttgggttg aggcctcttc tgcaggaagt ccctgagctg 2460
agacgcaagt tggctgggtg gtccacaccc tggctctcct gcaggtccac acaccttcca 2520
ggcctgtggc ctgcctccaa agatgtgcaa gggcaggctg gctgcacggg gagagggaag 2580
tattttgccg aaatatgaga actggggcct cctgctccca gggagctcca gggcccctct 2640
ctcctcccac ctggacttgg ggggaactga gaaacacttt cctggagctg ctggcttttg 2700
cactttttg atggcagaag tgtgacctga gagtcacacc ttctcttcag gaacgtagat 2760
gttggggtgt cttgccctgg ggggcttgga acctctgaag gtggggagcg gaacacctgg 2820
catccttccc cagcacttgc attaccgtcc ctgctcttcc caggtgggga cagtggccca 2880
agcaaggcct cactcgcagc cacttcttca agagctgcct gcacactgtc ttggagcatc 2940
tgccttgtgc ctggcactct gccggtgcct tgggaaggtc ggaagagtgg actttgtcct 3000
ggccttccct tcatggcgtc tatgacactt ttgtggtgat ggaaagcatg ggacctgtcg 3060
tctcagcctg ttggtttctc ctcattgcct caaaccctgg ggtaggtggg acgggggtc 3120
tcgtgcccag atgaaaccat ttggaaactc ggcagcagag tttgtccaaa tgaccctttt 3180
caggatgtct caaagcttgt gccaaaggtc acttttcttt cctgccttct gctgtgagcc 3240
ctgagatcct cctcccagct caaggacag gtcctgggtg agggtgggag atttagacac 3300
ctgaaactgg gcgtggagag aagagccgtt gctgtttgtt ttttgggaag agcttttaaa 3360
gaatgcatgt ttttttcctg gttggaattg agtaggaact gaggctgtgc ttcaggtatg 3420
gtacaatcaa gtgggggatt ttcatgctga accattcaag ccctccccgc ccgttgcacc 3480
cactttggct ggcgtctgct ggagaggatg tctctgtccg cattcccgtg cagctccagg 3540
ctcgcgcagt tttctctctc tccctggatg ttgagtctca tcagaatatg tgggtagggg 3600

gtggacgtgc acgggtgcat gattgtgctt aacttggttg tatttttcga tttgacatgg 3660

aaggcctgtt gctttgctct tgagaatagt ttctcgtgtc cccctcgcag gcctcattct 3720

ttgaacatca actctgaagt ttgatacaga taggggcttg atagctgtgg tcccctctcc 3780

cctctgacta cctaaaatca atacctaaat acagaagcct tggtctaaca cgggactttt 3840

agtttgcgaa gggcctagat agggagagag gtaacatgaa tctggacagg gagggagata 3900

ctatagaaag gagaacactg cctactttgc aagccagtga cctgcctttt gaggggacat 3960

tggacggggg ccgggggcgg gggttgggtt tgagctacag tcatgaactt ttggcgtcta 4020

ctgattcctc caactctcca ccccacaaaa taacggggac caatattttt aactttgcct 4080

atttgttttt gggtgagttt ccccccctcct tattctgtcc tgagaccacg ggcaaagctc 4140

ttcattttga gagagaagaa aaactgtttg gaaccacacc aatgatattt ttctttgtaa 4200

tacttgaaat ttattttttt attattttga tagcagatgt gctatttatt tatttaatat 4260

gtataaggag cctaaacaat agaaagctgt agagattggg tttcattgtt aattggtttg 4320

ggagcctcct atgtgtgact tatgacttct ctgtgttctg tgtatttgtc tgaattaatg 4380

acctgggata taaagctatg ctagctttca aacaggagat gcctttcaga aatttgtata 4440

ttttgcagtt gccagaccaa taaaatacct ggttgaaata caaaaaaaaa aaaaaaa 4497

<210> 9
<211> 5579
<212> DNA
<213> Homo sapiens

<400> 9
ggaccccggt gtctggcttc ccccgagccg ggaccccgcg atggccaagc gcagctcgct 60

gtacatccgc atcgtggagg ggaagaacct tcccgccaag gacatcactg gcagcagcga 120

cccctactgc atcgtgaagg tggacaatga gcccatcatc aggacagcca cagtgtggaa 180

gaccctgtgc cccttctggg gtgaggagta ccaagtgcac ctgccgccca ccttccacgc 240

tgtggctttc tatgtcatgg atgaggatgc cctcagccgg gacgacgtta tcggaaaggt 300

ctgccttaca agggacacca tagcctctca ccctaagggt ttcagcgggt gggcccacct 360

gacggaggtc gaccccgacg aggaggtgca gggcgagatc cacctgcggc tggaagtgtg 420

gccaggggcc cgggcctgcc ggctacgctg ctctgtgctg gaggccaggg atctggcccc 480

aaaggaccgc aatggcacat ctgacccctt cgtccgagtg cgctacaagg gccggacacg 540

ggagacctcg atcgtgaaga agtcatgcta cccacgctgg aatgagacgt ttgaatttga 600

gctgcaggag ggggccatgg aggcactgtg cgtggaggcc tgggactggg accttgtcag 660

ccgaaacgac ttcctgggca aagtggtgat tgatgtccag agactgcggg tggtgcagca 720

ggaggagggc tggttccggc tgcagcccga ccagtccaag agccggcggc atgacgaggg 780

caacctgggc tccttgcagc tggaggtgcg gctgcgggac gagacggtgc tgccctccag 840

ctactaccag ccactggtgc acctgctgtg ccacgaggtc aagctgggca tgcagggccc 900

agggcagctg atcccactca tcgaggagac aaccagcacc gagtgtcgcc aggacgtggc 960

cacgaacctg ctcaagctct tcctggggca ggggctggcc aaggacttcc tggacctgct 1020

cttccagctg gagctgagtc gcaccagtga gaccaacacc ctgttccgga gcaactctct 1080

ggcctcaaag tccatggagt cttttctgaa ggtggccggg atgcagtacc tgcacggcgt 1140

cctgggcccc atcatcaaca aggtgtttga ggagaagaag tacgtggagc tggaccccag 1200

caaagtggaa gttaaggatg tagggtgctc cgggctgcac cgcccgcaga ccgaggccga 1260

ggtgctggag cagagcgcgc agacgctgcg cgcccacctg ggggccctgc tgagcgcgct 1320

cagccgctcg gttcgcgcgt gccccgccgt ggtgcgcgcc accttccgcc agctcttccg 1380

gcgcgtgcgc gagcgcttcc ccggcgccca gcacgagaat gtaccgttca tcgccgtcac 1440

cagcttcctg tgcctgcgct tcttctctcc cgccatcatg tcgcccaagc tcttccacct 1500

gcgggagcgc cacgcggacg cccgcaccag ccgcaccctg ctcctgttgg ccaaggcagt 1560

ccagaacgtg ggcaacatgg acacgccggc ttccagggcc aaggaggctt ggatggagcc 1620

gctgcagccc accgtgcgcc agggcgtggc gcagctgaag gacttcatca ccaagctcgt 1680

ggacatcgag gagaaggacg agctggacct gcagcggacg ctgagtttgc aggcgccacc 1740

tgtgaaggag gggccactct tcatccacag gaccaagggc aagggccccc tcatgtcctc 1800

ctccttcaag aagctctact tctccctcac taccgaggcc ctcagcttcg cgaagatgcc 1860

cagctccaag aaaagcgccc tcatcaagtt agccaacatc cgggcagcgg aaaaggttga 1920

ggaaaagagc tttggcggct cgcacgtcat gcaggtcatc tacacggacg acgccggcag 1980

gccccagact gcctacctgc agtgcaagtg tgtgaatgag cttaaccagt ggctgtctgc 2040

gctgcggaag gtgagcatca acaacaccgg actgctgggc tcctaccacc ctggcgtctt 2100

ccgtggggac aagtggagct gctgccacca aaaagagaag acaggtcagg gctgcgataa 2160

gacccggtca cgggtgaccc tgcaggagtg gaatgaccct cttgaccatg accttgaggc 2220

ccagctcatc taccggcacc tgctgggcgt ggaggccatg ctgtgggaga ggcaccggga 2280

gctgagcggg ggcacagagg caggcacggt gcccacgagc cctggcaaag tccccgagga 2340

ctcattggcc cggctgctcc gggtgctgca ggacctccgc gaggcccata gctccagccc 2400

ggccggctcc ccaccctcag agcccaactg cctcctggag ctgcagacgt gaggcccgcc 2460

ctacgctccc cttgctgagt cccctgccaa gcgctcggag cccccccagg acactctgca 2520

ccccctcacc ccggtcctcc tcattagggt gcagggccta ggtctcttcc aggtggggga 2580

gggggagag tcaggaataa ggggatcccc agaagtgcag agctgagcag gcttgggcct 2640

gtcatggctg gccggaagtg tccccagctc cctacagacg ctgtagccat cactgcctct 2700

ccagggaccc tcctctcctg cccaggacag acccagccag aaccactgct aggatgggcc 2760

gcacccaggg gtctggcctc cagggaccta gagaatggga gggagaacgg ggccccagga 2820

gacccggccg ccaccccacc cgctaccctt gggtgccaca gggctgtgct gttgccaaca 2880

gtaaacctgc tcttactgtc caggctctgg ggtcttgtga tgagggtctg gggagaaagt 2940

gggcccgggg ggaccccgga ggctgtcggt ggatgtgccg atgatggggc tgacagtatg 3000

ggctctgggc atccctgttc cccccccttt cttcccccca ctcttctggg gtcggggtt 3060

cctttccctt cccagttgct gtccctgggt cccctctttc atgtcccaca ggccacagag 3120

cccagtgtgt ccaaccagct gttctctcct caaagcagcc cccaagcaag tcccttctct 3180

agggtgtccc tgaggacagc acagaggcgg gactcagaga ccccattcct cttcacgcag 3240

cccttacccc aagccctcta gctgtgtggc tggcagtgtt ggccacgtag ggctcccat 3300

ccccccacca ttgtgtcaca tgggctgcca ggctcagctc ccagctgcgt ccacagtgac 3360

ctggatcagg gtggggacaa ggactggacc ctccttctcc agaaggcctt cagctcttgc 3420

cttgccatgc agtcacctcc ttccccctct gaccccagat cccaaaggtg caccgttgcc 3480

ccagcccctt tctggcccca tggggtttct ctgatgcctt catcatagag gcccggggct 3540

ggtccgatgg ttggcaaaac ttgactccgg cccagtcccc actcttgggg acttagaacc 3600

cctgctgtcc tgggatctgg cctgcctttc tttggtcagt ccctgtggtc ccccaccagc 3660

tccccctccc atagggctgc ccaccaagcc ctgcccccag cccaagagga gcccccactg 3720

cctgcggggc agtgatgtct ggccaccggc tcacaccaat gacttggtcc tggggtggca 3780

gaagcagcag gtgacaggag cagggcccct gtccctctct tctggccctg tggtacccag 3840

gccacacgtt gtgcccgctc ttggggctga ccggctgtag ggaccaccag ccgctgctac 3900

tgtgggccgc cccggggcag ggtgggcagg gcttttgtgg gttatgagga cacagaagtc 3960

cctgaggccc ccagacctgg ctcagccaac ctccttcctc ccccggttgc cccccactct 4020

aaagcctcct ccctcccagc gtccactggc tccaggctcc tcacaacagc agctcataga 4080

cacggggcgt ctccaggtgg tcccagccct ccagatgttt ctagctctcc aggtgggcgc 4140

tgttttcacg tctgcctgca tccattcatt ccttcattcc tcacctttat cctgttatct 4200

ctattttttt aagctaccag gaaggaaagg gaagaagaga tcacgaaact gggaccccca 4260

gaagggagga gtgggctttg aacttagaca tctacctcag agctcaaata ggttgtttaa 4320

aatcacattc aattttcaga tgaaggggaa ctttatagtt tttttttttt tttttttttt 4380

ttttggggcg gagtctcact gtgttgccca ggctggagtg caaatggctt gatcttggtt 4440

cactgcaacc tctgcctccc aggttcaagc aattctcttg cctcggcctc ccgagtagct 4500

gggactggag gcgtgtgcca ccatgcccag ctaattcttg tatttttagt agagacggag 4560

tttctccatg ttggccagac tggtctcgaa ctcctgacct caggtgatct gaccgccttg 4620

gcctccgaaa gtgctgagat tacagttgcg agccactgtg cgtggccaga actttataat 4680

aagagacttg aagctgggtg tgacggtgca cgcctctggt cccagctgct cgggaggccg 4740

ggacagaagg atcaccttga ggccaggagt ttaaggccag cctgggcaac atagcaaaac 4800

ctagtcccta aaattaaaaa aaaaaaaaaa aaaaaaggaa aataaaggag acttgaaatt 4860

tttgaactaa atagtggtga tggctacaca ttgtgaatgt aattaacacc actgagttaa 4920

acacttaaaa tggttaaaat ggcaaattgt atgttatacc tattttacta caataaaaag 4980

tataaaaaag agaagatatt taggtgactt acagcaacca attgcaacaa aacaaaatgt 5040

taagaaatga tctttttatg aggcaattgg aaatttgaac actgatcaac tataggatga 5100

ttggaattat taatttttaa aggtgtgata agatgctgca cttggctggg cacagtggca 5160

catgcctgta atcccagcta cttggcaggc tgaggtggga gaatcgcttg agctcaggag 5220

ttcgagacca gcctgggcaa cgtggcgaaa tccccgtctt tacaaaaaca aacaaacaaa 5280

caaaaaagat attgcagttg tgttgtaagc gtccttatct ttcagagcta catagtggaa 5340

tgtttatgga atatttagga taaatgatat aggcatttgg gatttgctgc aaaatgaccc 5400

agaggcaggg gtcaggggga gaggtagaga tgagacaaga ggtagagggg agaggtagag 5460

gtagccacga gctgataatt acagacaaga gatgcggagt atgtgggggc tcattatcct 5520

gcatagtcta tctttgtata tctttgaact tttcaagaat aaaaaagctt aaaaagtat 5579

<210> 10
<211> 1207
<212> DNA
<213> Homo sapiens

<400> 10
acagagatca gcaaacagct tttctgagag aaagaaacat ctgcaaatga catgctgttt 60

ctgcagtttc tgctgctagc tcttcttctc ccaggtggtg acaatgcaga cgcatcccag 120

gaacacgtct ccttccatgt catccagatc ttctcatttg tcaaccaatc ctgggcacga 180

ggtcagggct caggatggct ggacgagttg cagactcatg gctgggacag tgaatcaggc 240

acaataattt tcctgcataa ctggtccaag ggcaacttca gcaatgaaga gttgtcagac 300

ctagagttgt tatttcgttt ctacctcttt ggattaactc gggagattca agaccatgca 360

agtcaagatt actcgaaata tccctttgaa gtacaggtga aagcgggctg tgagctgcat 420

tctggaaaga gcccagaagg cttctttcag gtagctttca acggattaga tttactgagt 480

ttccagaata caacatgggt gccatctcca ggctgtggaa gtttggccca aagtgtctgt 540

catctactca atcatcagta tgaaggcgtc acagaaacag tgtataatct cataagaagc 600

acttgccccc gatttctctt gggtctcctg gatgcaggga agatgtatgt acacaggcaa 660

gtgaggccag aagcctggct gtccagtcgc cccagccttg ggtctggcca gctgttgctg 720

gtttgtcatg cctccggctt ctacccaaag cctgtttggg tgacatggat gcggaatgaa 780

caggagcaac tgggcactaa acatggtgat attcttccta atgctgatgg gacatggtat 840

cttcaggtga tcctggaggt ggcatctgag gagcctgctg gcctgtcttg tcgagtgaga 900

cacagcagtc taggaggcca ggacatcatc ctctactggg gacaccactc ttccatgaat 960

tggattgcct tggtagtgat agtgcccttg gtgattctaa tagtccttgt gttatggttt 1020

aagaagcact gctcatatca ggacatcctg tgagactctt ccccctgact cccccattgt 1080

gttaagaacc cagcaaccca ggagcctagt acaatatagt gatgccatcc cgtcgactct 1140

ccatttaaat tgtttctctt tctgcataat aaacatttgt taataaaaac caaaaaaaaa 1200

aaaaaaa 1207

<210> 11
<211> 1925
<212> DNA
<213> Homo sapiens

<400> 11
   gagagggcga aggtaggctg gcagatacgt tcgtcagctt gctcctttct gcccgtggac 60
   gccgccgaag aagcatcgtt aaagtctctc ttcaccctgc cgtcatgtct aagtcagagt 120
   ctcctaaaga gcccgaacag ctgaggaagc tcttcattgg agggttgagc tttgaaacaa 180
   ctgatgagag cctgaggagc cattttgagc aatggggaac gctcacggac tgtgtggtaa 240
   tgagagatcc aaacaccaag cgctctaggg gctttgggtt tgtcacatat gccactgtgg 300
   aggaggtgga tgcagctatg aatgcaaggc cacacaaggt ggatggaaga gttgtggaac 360
   caaagagagc tgtctccaga gaagattctc aaagaccagg tgcccactta actgtgaaaa 420
   agatatttgt tggtggcatt aaagaagaca ctgaagaaca tcacctaaga gattattttg 480
   aacagtatgg aaaaattgaa gtgattgaaa tcatgactga ccgaggcagt ggcaagaaaa 540
   ggggctttgc ctttgtaacc tttgacgacc atgactccgt ggataagatt gtcattcaga 600
   aataccatac tgtgaatggc cacaactgtg aagttagaaa agccctgtca aagcaagaga 660
   tggctagtgc ttcatccagc caaagaggtc gaagtggttc tggaaacttt ggtggtggtc 720
   gtggaggtgg tttcggtggg aatgacaact tcggtcgtgg aggaaacttc agtggtcgtg 780
   gtggctttgg tggcagccgt ggtggtggtg gatatggtgg cagtggggat ggctataatg 840
   gatttggcaa tgatggtggt tatggaggag gcggccctgg ttactctgga ggaagcagag 900
   gctatggaag tggtggacag ggttatggaa accagggcag tggctatggc gggagtggca 960
   gctatgacag ctataacaac ggaggcggag gcggctttgg cggtggtagt ggaagcaatt 1020
   ttggaggtgg tggaagctac aatgattttg ggaattacaa caatcagtct tcaaattttg 1080
   gacccatgaa gggaggaaat tttggaggca gaagctctgg cccctatggc ggtggaggcc 1140
   aatactttgc aaaaccacga aaccaaggtg gctatggcgg ttccagcagc agcagtagct 1200
   atggcagtgg cagaagattt taattaggaa acaaagctta gcaggagagg agagccagag 1260
   aagtgacagg gaagctacag gttacaacag atttgtgaac tcagccaagc acagtggtgg 1320
   cagggcctag ctgctacaaa gaagacatgt tttagacaaa tactcatgtg tatgggcaaa 1380
   aaactcgagg actgtatttg tgactaattg tataacaggt tattttagtt tctgttctgt 1440
   ggaaagtgta aagcattcca acaaagggtt ttaatgtaga tttttttttt tgcaccccat 1500
   gctgttgatt gctaaatgta acagtctgat cgtgacgctg aataaatgtc ttttttttaa 1560
   tgtgctgtgt aaagttagtc tactcttaag ccatcttggt aaatttcccc aacagtgtga 1620
   agttagaatt ccttcagggt gatgccaggt tctatttgga atttatatac aacctgcttg 1680
   ggtggagaag ccattgtctt cggaaacctt ggtgtagttg aactgatagt tactgttgtg 1740
   acctgaagtt caccattaaa agggattacc caagcaaaat catggaatgg ttataaaagt 1800
   gattgttggc acatcctatg caatatatct aaattgaata atggtaccag ataaaattat 1860
   agatgggaat gaagcttgtg tatccattat catgtgtaat caataaacga tttaattctc 1920
   ttgaa 1925

<210> 12
<211> 8500
<212> DNA
<213> Homo sapiens

<400> 12
cgtcatggcc gtcagcaccg cgttcccgtc ctcttccgct tggccccaga aagtttcggt 60

tctgcccggc ggtggaccca cgagcgcgtg ccaccatgga gtctgaccac tgctgagcag 120

acagccaccg agggccgaaa ttctgagcct tcctctggac ccaggcagga gacatacaga 180

caagaaaggc aaactcacca tggcctccac caatgcagag agccagctcc agagaatcat 240

ccgagacttg caagatgctg tgacagaact aagcaaagaa tttcaggaag caggggaacc 300

catcacggat gacagcacca gcttgcataa attttcttat aaacttgagt atctcctgca 360

atttgatcag aaagagaagg ccaccctcct gggcaacaag aaggactact gggattactt 420

ctgtgcctgc ctggccaagg tgaaaggagc caatgatggg atccgatttg tcaagtctat 480

ctcagagctc cgaacatcct tggggaaagg aagagcattt attcgctact ccttggtgca 540

ccagaggttg gcagacacct tacagcagtg cttcatgaac accaaagtga ccagtgactg 600

gtactatgca agaagcccct ttctgcagcc aaagctgagc tcggacattg tgggccaact 660

ctatgagctg actgaggttc agtttgacct ggcgtcgagg ggctttgact tggatgctgc 720

ctggccaaca tttgccagga ggacgctgac cactggctct tctgcttacc tgtggaaacc 780

ccctagccgc agctccagca tgagcagctt ggtgagcagc tacctgcaga ctcaagagat 840
ggtgtccaac tttgacctga acagccccct aaacaacgag gcattggagg gctttgatga 900
gatgcgacta gagctggacc agttggaggt gcgggagaag cagctacagg agcgcatgca 960
gcagctggac agagagaacc aggagctgag ggcagctgtc agccagcaag gggagcaact 1020
gcagacagag agggagaggg ggcgcactgc agcggaggac aacgttcgcc tcacttgctt 1080
ggtagctgag ctccagaagc agtgggaggt cacccaggcc acccagaaca ctgtgaagga 1140
gctgcagaca tgcctgcagg ccctggagct aggagcagca gagaaggagg aggactacca 1200
cacagccctg cggcggctgg agtccatgct gcagcccttg gcacaggagc ttgaggccac 1260
acgggactca ctggacaaga aaaaccagca tttagccagc ttcccaggct ggctagccat 1320
ggctcagcag aaggcagatt cggcatcaga cacaaagggc cggcaagaac ctattcccag 1380
tgatgcggcc caggagatgc aggagctagg ggagaagctt caagccctag aaagggagag 1440
aaccaaggtc gaggaggtca acagacagca gagtgcccaa ctggaacagc tggtcaagga 1500
gcttcagctg aaagaggatg cccgggccag cctggagcgc ctggtgaagg agatggcccc 1560
actccaggag gagttgtctg ggaagggaca ggaggcagac cagctctggc gacggctgca 1620
ggagttgctg gcccacacga gctcctggga ggaggagcta gcagagttga ggcgggagaa 1680
aaaacagcaa caggaggaga aggagctgct ggagcaggag gtcaggtctc tgacccggca 1740
gctgcagttc ctggagaccc agctggcaca ggtgagccaa catgtgagtg acctggagga 1800
gcagaagaag cagctcattc aggacaaaga ccacctcagc cagcaggtgg gtatgctcga 1860
gcggcttgct gggccgcctg gcccagaact gccagtggca ggtgagaaga atgaggccct 1920
ggtccctgtg aactccagtc tgcaagaggc ctgggggaag ccagaggagg agcagagggg 1980
cctgcaggag gcacagttag acgataccaa ggtgcaagag ggcagccagg aggaagagct 2040
ccggcaggcc aacagggagc tggagaagga gctacagaat gtggtcgggc gtaaccagct 2100
cctggagggc aagctgcaag ccctgcaggc cgattaccag gctttgcagc agcgggaatc 2160
agccatccag ggctccttgg cctccctgga ggccgagcag gccagcatcc ggcacttggg 2220
tgaccagatg gaggcgagct tgctggctgt aaggaaggcc aaggaggcca tgaaagccca 2280
gatggcagag aaggaggcca ttctacagag caaggagggc gagtgtcagc agctgcggga 2340
ggaggtggag cagtgccagc aactggcaga agcccggcac agagagctta gggctctcga 2400
gagccagtgc cagcagcaga cccagctgat tgaggtcctc acagcagaga aaggccaaca 2460
gggagttggc ccacccactg acaatgaagc ccgtgagctg gctgcccagc tagccctgtc 2520
tcaggcgcag ctggaagtcc atcaggggga ggtccaacgg ctgcaggctc aggtggtgga 2580
cctccaggcc aagatgcggg cagccctgga tgaccaggac aaggtgcaga gccagctaag 2640

catggctgag gccgtcctga gggagcacaa aacccttgtg cagcagctga aggagcagaa 2700

tgaagccctt aacagagccc atgtccagga gctgctgcaa tgctcggagc gtgaaggggc 2760

actgcaggag gagagggccg atgaggccca gcagagggag gaggagctgc gggccctgca 2820

ggaggagctg tcccaggcca aatgcagctc cgaggaagca cagctggagc acgctgagct 2880

gcaagagcag ctgcaccggg ccaacacaga cacagctgag ctgggcatcc aggtttgcgc 2940

actgaccgtg gaaaaggagc gagtggagga ggcactggcc tgtgctgtcc aggagctcca 3000

ggacgccaaa gaggcagcct caagggagcg agagggcctg gagcgccaag tagctgggct 3060

gcagcaagag aaggagagct tgcaggagaa gctgaaggcg gccaaggcag cagccggctc 3120

actgcctggc ctgcaggccc agctcgccca ggcagagcag cgggcccaga gcctccaaga 3180

ggctgcacac caggagctca acaccctcaa gttccagctg agtgctgaaa tcatggacta 3240

ccagagcaga cttaagaatg ctggtgaaga gtgcaagagc ctcaggggcc agcttgagga 3300

gcaaggccgg cagctgcagg ctgctgagga agctgtggag aagctgaagg ccacccaagc 3360

agacatggga gagaagctga gctgcactag caaccatctt gcagagtgcc aggcggccat 3420

gctgaggaag gacaaggagg gggctgccct gcgtgaagac ctagaaagga cccagaagga 3480

actcgaaaaa gccacaacaa aaatccaaga gtattacaac aaactctgcc aggaggtgac 3540

aaatcgtgag aggaatgacc agaagatgct tgctgacctg gatgacctca acagaaccaa 3600

gaagtatctc gaggagcggc tgatagagct gctcagggac aaggatgctc tctggcagaa 3660

gtcagatgcc ctggaattcc agcagaagct cagtgctgag gagagatggc tcggagacac 3720

agaggcaaac cactgcctcg actgtaagcg ggagttcagc tggatggtgc ggcggcacca 3780

ctgcaggata tgtggccgca tcttctgtta ctactgctgc aacaactacg tcctgagcaa 3840

gcacggtggc aaaaaggagc gctgctgccg agcctgtttc cagaagctca gtgaaggccc 3900

tggctcccct gatagcagtg gctcaggcac tagccaggga gagcccagcc ctgcactgtc 3960

accagcctca cctgggcccc aggccacagg aggccaagga gcaaatacag actacaggcc 4020

accggacgac gctgtgtttg atatcatcac agatgaggaa ttgtgccaga tacaggagtc 4080

cggctcctct ttgcctgaaa cacccactga aactgattct cttgacccaa atgcggctga 4140

acaggatact acatcaacct cgctaacgcc tgaggacact gaagacatgc ccgtggggca 4200

ggattcggaa atctgcctgc tgaagtctgg agaactgatg atcaaagtac ccctcacagt 4260

ggatgagatc gccagcttcg gggagggtag cagggagctg tttgtgaggt ccagcaccta 4320

cagcctgatc cccatcactg tgcccgaggc agccctcacc atcagctggg tcttctcctc 4380

tgaccccaag agcatctcct tcagtgtggt cttccaggag gccgaggaca caccgctgga 4440

tcagtgtaag gtcctcattc ccacgacccg atgcaactcc cacaaggaga acatccaggg 4500

ccagctcaag gttcgcacac ccggcatcta catgctcatc ttcgacaata ccttctcaag 4560

gtttgtctct aaaaaggtat tttatcactt gacggttgat cggcctgtga tctacgatgg 4620
aagtgatttc ctgtagcttc agcacctcag taacttcact tcatccacag gaaacactgc 4680
tcttcctcac ctgtcacata aagcattttt ttaaaaagtc agctgctcca aaatcatcaa 4740
ctcagcccct gggctgcccc tcagaggcgg tgtctgggga ggactttgtg ctcagcactc 4800
tgcaccggcc actcttagcc cccgaggcgt tgaagggctc aggcaatgtt tccattaagt 4860
agagactcag ctgttgtcac acccaaaggg atgctctgcc aaaggtttaa acacccagga 4920
gaccatcagc ctctcctggg agcacagttg actacaggcc tcttgtggag agtttcacgg 4980
gcaggggtga ttccaacttc tgcctgtgga gagattttct gccctgcccc accagggccc 5040
tgcatgttgg agactgagct gggtgcactg gccataccct gtgaatcctc gggctgtgac 5100
gccctcaggt actcctggga aaaggaggta cacagccatc atgcgagtcg gtgccagggg 5160
accccccgga gatcctgacc agctcctcca gtcatgctct tgtccctcac tgccccagta 5220
agctggaggc tgctccagaa ctcagcagtg ttggaggggc ctctaagctg cactctcttt 5280
ctggcccttt tgtctggctg attctgtcct caaataaagc ccttcactca gccagacctc 5340
tccacagctc aaagcattgc cctaagaatc agaagtaaag ataatccaag agcaaaaccc 5400
actgtacttg gggcctgcaa tggctgtgtg tacactacat ctaatgccca aatgccagcc 5460
agtgtggatg ttgtgaccac agagcaggat tgtgcattgg ctttagagct actcctcagc 5520
tgatggccca cttttgttta tataaataag agcttctgcc ccacctgcag acatgtttac 5580
taatgatcat agccaggatt agaaccactt tcaaacattg gggccttctt aacaaaagtc 5640
tttgataact taagaaccaa agtaacagag taaacagagg catgatggat ccctgggccc 5700
cactcccctc ctgacaggtt ccccaacagc ccatttgccc acttcccact gctcagccca 5760
caccagacct ccaggagaca tccccccttg aggcagagag atcctgttcc ctattcccag 5820
acaagaatta tttaatcttc cctgttctct gtggtccttt tcttccccaa caacagatag 5880
ctcaccttgg acagctcttc gtcccttgtt catggaacca gctgcctgca gtcaggcccc 5940
aggttcttcc atgggtgaac agagcatctg acaaaaggtc ccagtttggc caggggtgag 6000
ggagagagca ccagacaggc tatccgagaa tctgagagct gggcccggca attcctccag 6060
ctacccttgt gacctaagtc cagtcacaca tttcccaaag tttctctttg tcataaccct 6120
ggtctggctg gttttgaggg cttgagaatg ggtcagggac tccaggccaa gtccaacaga 6180
gaccccaaac ccaccacaca ccagcagcca caacctcacc accaacaaag aggacttttg 6240
tggggccaca agtaagaggt catttctgga atggactcag acctttaaac aggagagttg 6300
agcacttcca gtcagttttt aagcaaggca tggggaacag ggaatagaac ctttcaaaga 6360
ggttgcccag agaaaagctg ggcctcttgc attcggcttc cttggagcag cctcttctgg 6420

cagaaagcca tcaggtgctc aatcatcttc tcctggccaa ggctctgacc atgcttagta 6480

ctggaataga ggtggccagg cccccagcga ctcttcttgg cctgatgttt gtcctcacag 6540

gcatgccacg tggcctgaga tgattcagaa caaatcatgc taactttgaa tccatccagc 6600

cacttgcaaa tgataatcag aagtcagctt gttcactgtt agaaagaaac taacaaaaga 6660

gaacccagag caatctagaa tctttgagtg cttggctttc caaggatact gcggagactc 6720

tggccaagct gatgaccttc tgaagtgtca ctggcaccat atgcaacaag aaccaccatt 6780

cactgagtag ctaatgggtt tggggcctgg gacattccat ctgaggtcct tcctgaacat 6840

gtcactccac agcagaggac cggttgcagc ttacccagaa ccactcctcc aggagagctg 6900

gatgttttgc gtgcaacacc ttgagcactg actgctattg ttcaaaaaaa gcctttgctg 6960

cattcggagg actgccccgt gccctgaggt gacttcctaa ctatgtggtt tcattagcga 7020

atttattttt tgggctgggt ggacatttgt attttgttag gttgctgttt aagctcaagt 7080

ttgctgtgct ctctgcagct acaaaacatc ttggcatatt taagagtggc ttttataaat 7140

agctttattc tgatattaat cagattccca actttactga gaattaagga ctggggtact 7200

ttaaagaaat gcaaatagca attgaagaac cactgctgca ggtggtagcc ctggctagac 7260

tgaattacac tagaaatcag ccagaaggaa gcgtccttgg gatcccagat cactcttttt 7320

tttttttttt tttaaaaggg gcagcccctt gatggctcat ctctctgaat aacagttacg 7380

tcttcatatc gataccagat gccttcttca tcatgccact gaagccactc accaccttca 7440

agaacatgcc aacctctgtc agattcactt acccacaaac aaggaggcac gtttggcaca 7500

aagtgttgtc ctccaggtcc aagtggactc tacagagtgc ttgacctcaa cacactggat 7560

tccaggtgga ctggaccaag agcaggcaaa gacacgggaa ctgaaaaact ccacagggtt 7620

tggagaatag aaatgaaaag ccacgtcata taactcaaga ataaatggtg ttttggaaat 7680

tttaaaatta tcatcgaagg tggtgaaact atttcaggcc caaatgaaag gaaatcgcca 7740

gttggggatg aaatcacaga gcctgtgttt tatgatatgg ttggatgtcc actgatgaaa 7800

ttttaaagga gtttcatttt taaaagtgcg catgattcta catatgagaa ttctttaggc 7860

caagaaactg tccttggctc agaggtgttg ggaattaaag cagagagaag ccattcgtga 7920

tgcttagaac caaggatggt catgtacaca aagaccatcg agacggccat tcttgtttac 7980

aaaacactta ccaagaaagc actttgtagg ggaactttag taagttcttc tcatttcatt 8040

atgtttcttc caaggaaaca ggagagactg aattaataat tctctctttc ctcttaagca 8100

cttttaaaat aataaagtac atcttgaaat ttggggaggc atctctgatt taaaaaaaga 8160

aaaaggctgc ttgatgtatg ttatgcagag acactctgcc tctggtggct gcagagcaat 8220

acccaagcct catttggaag gctcaacatt tggaattgca ctttaattga ttaatcctca 8280

attcatgtgg ccttacggga tggtgggtct gggaccccaa ttcattctta tctgccaaag 8340

aattatctag aagcacatca aataccagca ccccacctgc acaatggggg tggaaaactt 8400

ttgtatccct aagcatatta ttttatagtg tctgccatgc catgtggaaa tactttattt 8460

ttaacctcag gatttaaata aagtaaacac tatgacattt 8500

<210> 13
<211> 5010
<212> DNA
<213> Homo sapiens

<400> 13
ggcggctcgg gacggaggac gcgctagtgt gagtgcgggc ttctagaact acaccgaccc 60

tcgtgtcctc ccttcatcct gcggggctgg ctggagcggc cgctccggtg ctgtccagca 120

gccataggga gccgcacggg gagcgggaaa gcggtcgcgg ccccaggcgg ggcggccggg 180

atggagcggg gccgcgagcc tgtggggaag gggctgtggc ggcgcctcga gcggctgcag 240

gttcttctgt gtggcagttc agaatgatgg atcaagctag atcagcattc tctaacttgt 300

ttggtggaga accattgtca tatacccggt tcagcctggc tcggcaagta gatggcgata 360

acagtcatgt ggagatgaaa cttgctgtag atgaagaaga aaatgctgac aataacacaa 420

aggccaatgt cacaaaacca aaaaggtgta gtggaagtat ctgctatggg actattgctg 480

tgatcgtctt tttcttgatt ggatttatga ttggctactt gggctattgt aaaggggtag 540

aaccaaaaac tgagtgtgag agactggcag gaaccgagtc tccagtgagg gaggagccag 600

gagaggactt ccctgcagca cgtcgcttat attgggatga cctgaagaga aagttgtcgg 660

agaaactgga cagcacagac ttcaccagca ccatcaagct gctgaatgaa aattcatatg 720

tccctcgtga ggctggatct caaaaagatg aaaatcttgc gttgtatgtt gaaaatcaat 780

ttcgtgaatt taaactcagc aaagtctggc gtgatcaaca ttttgttaag attcaggtca 840

aagacagcgc tcaaaactcg gtgatcatag ttgataagaa cggtagactt gtttacctgg 900

tggagaatcc tgggggttat gtggcgtata gtaaggctgc aacagttact ggtaaactgg 960

tccatgctaa ttttggtact aaaaaagatt ttgaggattt atacactcct gtgaatggat 1020

ctatagtgat tgtcagagca gggaaaatca cctttgcaga aaaggttgca aatgctgaaa 1080

gcttaaatgc aattggtgtg ttgatataca tggaccagac taaatttccc attgttaacg 1140

cagaactttc attctttgga catgctcatc tggggacagg tgacccttac acacctggat 1200

tcccttcctt caatcacact cagtttccac catctcggtc atcaggattg cctaatatac 1260

ctgtccagac aatctccaga gctgctgcag aaaagctgtt tgggaatatg gaaggagact 1320

gtccctctga ctggaaaaca gactctacat gtaggatggt aacctcagaa agcaagaatg 1380

tgaagctcac tgtgagcaat gtgctgaaag agataaaaat tcttaacatc tttggagtta 1440

ttaaaggctt tgtagaacca gatcactatg ttgtagttgg ggcccagaga gatgcatggg 1500

gccctggagc tgcaaaatcc ggtgtaggca cagctctcct attgaaactt gcccagatgt 1560
tctcagatat ggtcttaaaa gatgggtttc agcccagcag aagcattatc tttgccagtt 1620
ggagtgctgg agactttgga tcggttggtg ccactgaatg gctagaggga tacctttcgt 1680
ccctgcattt aaaggctttc acttatatta atctggataa agcggttctt ggtaccagca 1740
acttcaaggt ttctgccagc ccactgttgt atacgcttat tgagaaaaca atgcaaaatg 1800
tgaagcatcc ggttactggg caatttctat atcaggacag caactgggcc agcaaagttg 1860
agaaactcac tttagacaat gctgctttcc ctttccttgc atattctgga atcccagcag 1920
tttctttctg tttttgcgag gacacagatt atccttattt gggtaccacc atggacacct 1980
ataaggaact gattgagagg attcctgagt tgaacaaagt ggcacgagca gctgcagagg 2040
tcgctggtca gttcgtgatt aaactaaccc atgatgttga attgaacctg gactatgaga 2100
ggtacaacag ccaactgctt tcatttgtga gggatctgaa ccaatacaga gcagacataa 2160
aggaaatggg cctgagttta cagtggctgt attctgctcg tggagacttc ttccgtgcta 2220
cttccagact aacaacagat ttcgggaatg ctgagaaaac agacagattt gtcatgaaga 2280
aactcaatga tcgtgtcatg agagtggagt atcacttcct ctctccctac gtatctccaa 2340
aagagtctcc tttccgacat gtcttctggg gctccggctc tcacacgctg ccagctttac 2400
tggagaactt gaaactgcgt aaacaaaata acggtgcttt taatgaaacg ctgttcagaa 2460
accagttggc tctagctact tggactattc agggagctgc aaatgccctc tctggtgacg 2520
tttgggacat tgacaatgag ttttaaatgt gatacccata gcttccatga gaacagcagg 2580
gtagtctggt ttctagactt gtgctgatcg tgctaaattt tcagtagggc tacaaaacct 2640
gatgttaaaa ttccatccca tcatcttggt actactagat gtctttaggc agcagctttt 2700
aatacagggt agataacctg tacttcaagt taaagtgaat aaccacttaa aaaatgtcca 2760
tgatggaata ttcccctatc tctagaattt taagtgcttt gtaatgggaa ctgcctcttt 2820
cctgttgttg ttaatgaaaa tgtcagaaac cagttatgtg aatgatctct ctgaatccta 2880
agggctggtc tctgctgaag gttgtaagtg gttcgcttac tttgagtgat cctccaactt 2940
catttgatgc taaataggag ataccaggtt gaaagacctc tccaaatgag atctaagcct 3000
ttccataagg aatgtagcag gtttcctcat tcctgaaaga aacagttaac tttcagaaga 3060
gatgggcttg ttttcttgcc aatgaggtct gaaatggagg tccttctgct ggataaaatg 3120
aggttcaact gttgattgca ggaataaggc cttaatatgt taacctcagt gtcatttatg 3180
aaaagagggg accagaagcc aaagacttag tatattttct tttcctctgt cccttccccc 3240
ataagcctcc atttagttct ttgttatttt tgtttcttcc aaagcacatt gaaagagaac 3300
cagtttcagg tgtttagttg cagactcagt ttgtcagact ttaaagaata atatgctgcc 3360

aaattttggc caaagtgtta atcttagggg agagctttct gtccttttgg cactgagata 3420

tttattgttt atttatcagt gacagagttc actataaatg gtgttttttt aatagaatat 3480

aattatcgga agcagtgcct tccataatta tgacagttat actgtcggtt ttttttaaat 3540

aaaagcagca tctgctaata aaacccaaca gatactggaa gttttgcatt tatggtcaac 3600

acttaagggt tttagaaaac agccgtcagc caaatgtaat tgaataaagt tgaagctaag 3660

atttagagat gaattaaatt taattagggg ttgctaagaa gcgagcactg accagataag 3720

aatgctggtt ttcctaaatg cagtgaattg tgaccaagtt ataaatcaat gtcacttaaa 3780

ggctgtggta gtactcctgc aaaattttat agctcagttt atccaaggtg taactctaat 3840

tcccatttgc aaaatttcca gtacctttgt cacaatccta acacattatc gggagcagtg 3900

tcttccataa tgtataaaga acaaggtagt ttttacctac cacagtgtct gtatcggaga 3960

cagtgatctc catatgttac actaagggtg taagtaatta tcgggaacag tgtttcccat 4020

aattttcttc atgcaatgac atcttcaaag cttgaagatc gttagtatct aacatgtatc 4080

ccaactccta taattcccta tcttttagtt ttagttgcag aaacattttg tggtcattaa 4140

gcattgggtg ggtaaattca accactgtaa aatgaaatta ctacaaaatt tgaaatttag 4200

cttgggtttt tgttaccttt atggtttctc caggtcctct acttaatgag atagcagcat 4260

acatttataa tgtttgctat tgacaagtca ttttaattta tcacattatt tgcatgttac 4320

ctcctataaa cttagtgcgg acaagtttta atccagaatt gaccttttga cttaaagcag 4380

agggactttg tatagaaggt ttgggggctg tggggaagga gagtcccctg aaggtctgac 4440

acgtctgcct acccattcgt ggtgatcaat taaatgtagg tatgaataag ttcgaagctc 4500

cgtgagtgaa ccatcatata aacgtgtagt acagctgttt gtcatagggc agttggaaac 4560

ggcctcctag ggaaaagttc atagggtctc ttcaggttct tagtgtcact tacctagatt 4620

tacagcctca cttgaatgtg tcactactca cagtctcttt aatcttcagt tttatcttta 4680

atctcctctt ttatcttgga ctgacattta gcgtagctaa gtgaaaaggt catagctgag 4740

attcctggtt cgggtgttac gcacacgtac ttaaatgaaa gcatgtggca tgttcatcgt 4800

ataacacaat atgaatacag ggcatgcatt ttgcagcagt gagtctcttc agaaaaccct 4860

tttctacagt tagggttgag ttacttccta tcaagccagt acgtgctaac aggctcaata 4920

ttcctgaatg aaatatcaga ctagtgacaa gctcctggtc ttgagatgtc ttctcgttaa 4980

ggagtagggc cttttggagg taaaggtata 5010

<210> 14
<211> 3835
<212> DNA
<213> Homo sapiens

<400> 14

cgggcgcgcc gggagggcgc gaggtcaggg ggctggggac gcgcgtgggg atcgctaccc 60
ggctcggcca ctgctgggcg gacacctggg cgcgccgccg cgggaggagc ccggactcgg 120
gccgaggctg cccaggcaat gcgttcactc ggcgcaaaca tggctgcggc cctgcgcgcc 180
gcgggcgtcc tgctccgcga tccgctggca tccagcagct ggagggtctg tcagccatgg 240
aggtggaagt caggtgcagc tgcagcggcc gtcaccacag aaacagccca gcatgcccag 300
ggtgcaaaac ctcaagttca accgcagaag aggtatgagt ctaacatcag gaagccgaaa 360
actggaatat taatgctaaa catgggaggc cctgaaactc ttggagatgt tcacgacttc 420
cttctgagac tcttcttgga ccgagacctc atgacacttc ctattcagaa taagctggca 480
ccattcatcg ccaaacgccg aacccccaag attcaagagc agtaccgcag gattggaggc 540
ggatccccca tcaagatatg gacttccaag cagggagagg gcatggtgaa gctgctggat 600
gaattgtccc ccaacacagc ccctcacaaa tactatattg gatttcggta cgtccatcct 660
ttaacagaag aagcaattga agagatggag agagatggcc tagaaagggc tattgctttc 720
acacagtatc cacagtacag ctgctccacc acaggcagca gcttaaatgc catttacaga 780
tactataatc aagtgggacg gaagcccacg atgaagtgga gcactattga caggtggccc 840
acacatcacc tcctcatcca gtgctttgca gatcatattc taaaggaact ggaccatttt 900
ccacttgaga agagaagcga ggtggtcatt ctgttttctg ctcactcact gcccatgtct 960
gtggtcaaca gaggcgaccc atatcctcag gaggtaagcg ccactgtcca aaaagtcatg 1020
gaaaggctgg agtactgcaa cccctaccga ctggtgtggc aatccaaggt tggtccaatg 1080
ccctggttgg gtcctcaaac agacgaatct atcaaagggc tttgtgagag ggggaggaag 1140
aatatcctct tggttccgat agcatttacc agtgaccata ttgaaacgct gtatgagctg 1200
gacatcgagt actctcaagt tttagccaag gagtgtggag ttgaaaacat cagaagagct 1260
gagtctctta atggaaatcc attgttctct aaggccctgg ccgacttggt gcattcacac 1320
atccagtcaa acgagctgtg ttccaagcag ctgaccctga gctgtccgct ctgtgtcaat 1380
cctgtctgca gggagactaa atccttcttc accagccagc agctgtgacc cccgccggtg 1440
gaccccgtgg cgttaggcaa atgcccaacc tccagatacc tccgatgtgg agagggtgtt 1500
atttagagat caaggaagga agtcatcctt ccttgatata tatacagcct ttgggtacaa 1560
attgtgtggt ttcttgagga ttggactctt gatggatttc tatttttata taactataca 1620
gtaagcattt gtattttctc tctctaggta taagttacta gtttggaatg tccatcagga 1680
cctttaataa atgagttaaa aatttgtctt atgagacaca cctatttaag tacagatttt 1740
ggctttattg cccaaaaccc tcctgaaagg gtacggagag tcccctctgt gggctggcag 1800
tgtgaatgag atctgtttag tctcgtgcat atagttgctg tttttaaat gaacacagtt 1860
gagtatttga agtgaatttg aaaaagaaat gttacttaat ctttccctaa gcccatgggt 1920

tacagaatgc tagggaggca atttggttac ctgcaatggc tgcttttgcc agcgaggcca 1980
ccattcattg gtcatcttgg tatttgtgtg tgaatctcac tttcctcaat gtaaaaagga 2040
atcaagtatg gatttcagag gtgctcttag attccccata cacccaaggg taataaacgt 2100
gtacaagtac agtgttcatg atacgtgcct tggtgggagt ccgtggtgcc acagggaagg 2160
ggctcccact gcttctggtc tccaggaca gtgctgctgg aaaggctagt gatgagcttc 2220
accctggagc tcctcccggg accttgcaag cctctccatc cagcatcttc tctatcttag 2280
ttgaatgcct tctttctgaa catttgtttt aagaattatt ttataaagtc aacaatactt 2340
tgcttgaatt ctttcttaat ttacgatttt ttattataaa aatgtatagt gatacaatgg 2400
gacatgtgaa gaatacagaa aagtaaccac tttaatgcaa taactgttat cataatattg 2460
tatttcgtgg tagtcctttc ctgtagatat ttttaatgcc atttaatgcc attgtcacct 2520
tggatttatg agtgaaaagt gtttctaaaa atatagaaat aatgtcagat cagagtctga 2580
tcttctatgt ttgtatttaa atggattaaa agatccccgg tggttccatg aagaatttgt 2640
aaagatcact ttctctttcc tccaagccct gaaactttgt tcttcaaaag agcgtttctt 2700
tttttttttt tttttagcca gtttataaag tggaagtatt aggagattca taaatcttct 2760
atattgagaa ttggctatgt taataaatat tacaacatca ttaaggtttt agctaagttt 2820
gattcatgct gtctgttaaa tcaaaactga tctaaatcag aattattaaa tgtgaggagc 2880
tttttttaata caggaaaaga aacatgtcat ccacttgagt taatagtttt cctacgttga 2940
tgacagccct catgagtagc atccacattt ttaaaatttc aaattggttt ttctactagt 3000
agattgtgtt tctagagaaa gatacaaggc ataggtgatt gtttaggatt ttcctctagc 3060
ctttgccatt acctttttgg ggatgaggtt cacagtagac tttgagtgac cgtcccaccg 3120
tgaagtgaat tctctgagct ggtggtgtgg tgctggaagg aaggttattt ttggagccac 3180
tctctcccct taaggatatt tcccaagggc ctgcttcaat tctttgatga ctttagaggt 3240
gaaaaaatat ttttatggag atgatgcaga aaactccaat tcaggagccc ttgcgagtat 3300
atctgaagca cttatttgct aaggaaacct gaattgatag cagtactgtg ctgtctggaa 3360
taatgtcctt gatactgagt tgggaccaga ctggctttta tagtgacagg caaagaggaa 3420
tttattgaga tcactgctca tggcatttgt tgctgtaaga agtgttgcct ttgattgtta 3480
ctaaccacgg atgggtaacg gtcatacatt aggctagtgt ttggtaggac aaaatctttt 3540
tagagctttg agaattgtca tcctgttggt caactttgaa atacaaatgt ttgccctggt 3600
aattagcaat gaactgctgg cagtttcttc agctgtgtat atacggatct ggcttttaat 3660
tgatgaatca acttctacag aaacttttgc agggacagtg ttgatgaggc agtttagctt 3720
gccagggtga tgataaagcc caggtccctg catgtatagt gctcttctaa agaatatgca 3780

ttcttgaact acttaacttt ttaaaaatca caataaattt ttgcactcaa aattt 3835

<210> 15
<211> 2745
<212> DNA
<213> Homo sapiens

<400> 15
aggacagccg gcgcgcggcc gtgcccacaa gttgccggca gctgagcgcc gcgcctcctc 60

ctgctcgcag ccccctacgc ccacccggcg gcggtggcca gcgccaggac gcacatcccg 120

cggacaccga ccccagatgt aaagcgggac cccagcccct cgcccccggg cgcgatcgac 180

agtctcgcca gcgtctcctc tgccaaaacc cagggctgga agatgtggca gccggccacg 240

gagcgcctgc aggagagatt tgcagacaca gaagcggcac agagaaggcc attgtgaaga 300

tcaaggcaga aaccggagtt atggcatcat aagccaagga atgccaagga ttgctggcaa 360

ccacctgatg ttagaagagt cgaggacatg ttcttctcca gagcttttgg atggtgtgtg 420

gccctgccaa cctttacatt ttggacttcc agcctccgaa atgcactttc agaccatgct 480

gaagtctaaa ttgaatgtct taacactgaa aaaggaacct ctcccagcgg tcatcttcca 540

tgagccggag gccattgagc tgtgcacgac cacaccgctg atgaagacaa ggactcacag 600

tggctgcaag gttacctacc tgggcaaagt ctccaccact ggcatgcagt ttttgtcagg 660

ctgcacagaa aagccagtca ttgagctctg gaagaagcac acgctagccc gagaggatgt 720

ctttccggcc aatgccctcc tggaaatccg gccattccaa gtttggctcc atcatctcga 780

ccacaaaggg gaggccacag tgcacatgga taccttccag gtggcccgca tcgcctactg 840

caccgccgac cacaacgtga gccccaacat cttcgcctgg gtctacaggg agatcaatga 900

tgacctgtcc taccagatgg actgccacgc cgtggagtgc gagagcaagc tcgaggccaa 960

gaaactggcc cacgccatga tggaggcctt caggaagact ttccacagta tgaagagcga 1020

cgggcggatc cacagcaaca gctcctccga agaggtttcc caggaattgg aatccgatga 1080

tggctgaatg aacttgagac gcttcagcaa aggcagcatt ggtcacggag ttcaagggaa 1140

tagatgagta agcaacgttt caaatttggg atgaaaagac tgccaaacta ttggctgacc 1200

aaggttttta aattcagaag agcaattcta aatctaaaga aatgtatcat taaagtaatt 1260

acgttacatt gaaacctgct gctgctgtga ctgtgaggag ggtgggagtg tggatgggga 1320

ggaaggttct aggctctctt atttttctca tttcccaatg cctctctgtg ggagagctcc 1380

atgccagttt tcaccacgct caggcaaata ctctgcagct gttattggat gggccattcc 1440

gatctgcctt atgaaattcc acaagaatgt taggggcacc tatgggatct ctagtggggt 1500

gggcagggtg ctgatgggga cgctggccgc agggaggaag gaacatctcg ggagggccct 1560

ctgttcctct cccacggcag atgccctcct ctgtatgcaa atcagcacag cctttattga 1620

gctttacaac taacaacctg atagttggca gttaattcac agttacagat aatgctttta 1680

tttacataaa tataccaagt agtaccctct tattgtattc acttcatcta ttttcttaga 1740

atacttgcaa ttactaatga ccccttccct ttccctcctg ctgccctgtc caccctcttt 1800

ccccttctaa catccttaga gggatgaaat ctcagcatat gttgcaggac accaaaagga 1860

agaaaacaat caagcaaata aaataaacag tcaaacaaac caggagttta aaacaacaac 1920

cccaacaaca gaagccttgg caaagaggaa taagtgatca gcaagtgaac acactctatg 1980

tcaactctcc ttttatccag ctgagattta tggtaactta tttaattaat ggtcctgtct 2040

gatgcatcct tgatggcaag cttcaaatct gatttgctat caccgaggaa accttgcccc 2100

catcactcag cattgcactt agatacagaa tgagttagat aaacttggct tgtctagaga 2160

cccatgtcat cttaacctaa agggaaatct tattgcgtta tcataaaatt gatgatatct 2220

tagggtcaga attgcccttt ttttttattt tgaatgggaa gttctcacta aaacaatcct 2280

gagatttctt aatttcatgg ttctttaaat attataaaca cagagtcaac atagaatgaa 2340

attgtatttg ttaaaataca cacattggag gacaagagca gatgactact tttcgaagta 2400

atgctgctcc ttcctaaaag tctgttttca atcctggtaa tattaggggc actgcggcac 2460

ctaagaagcc ttaaatgaga gctaatccaa tctagagagc gatggtgtca gcatttcggt 2520

ctgcatatct gtgtgtccgt atctgcgttt gtgtgcgtgt acgtgtgccc ctgtgtgtgg 2580

gcccagtttt caggcatgta gaataagcat ggagtcatat tgaggaggac tcacttcttg 2640

aagatatgct tgttgcttta caacatatgt aagctattct ttagcataaa tgcattcatt 2700

ctttaataaa aatatgtttg cattaataaa gctgaggagt ttcat 2745

<210> 16
<211> 3689
<212> DNA
<213> Homo sapiens

<400> 16
cggccgctct cgtcgccgcc gcagcgggcg cgtcggaggg agcccagcat ggccgggccg 60

ggctcgccgc gccgcgcgtc ccgggggccc tcggcgcttc tcgctgccgc gcttctctac 120

gccgcgctgg gggacgtggt gcgctcggag cagcagatac cgctctccgt ggtgaagctc 180

tgggcctcgg cttttggtgg ggagataaaa tccattgctg ctaagtactc cggttcccag 240

cttctgcaaa agaaatacaa agagtatgag aaagacgttg ccatagaaga aatcgatggc 300

ctccaactgg taaagaagct ggcaaagaac atggaagaga tgtttcacaa gaagtctgag 360

gccgtcaggc gtctggtgga ggctgcagaa gaagcacacc tgaaacatga atttgatgca 420

gacttacagt atgaatactt caatgctgtg ctgataaatg aaaggacaa agacggaat 480

tttttggagc tgggaaagga attcatctta gccccaaatg accattttaa taatttgcct 540

gtgaacatca gtctaagtga cgtccaagta ccaacgaaca tgtacaacaa agaccctgca 600
attgtcaatg ggtttattg gtctgaatct ctaaacaaag tttttgtaga taactttgac 660
cgtgacccat ctctcatatg gcagtacttt ggaagtgcaa agggcttttt taggcagtat 720
ccggggatta aatggaacc agatgagaat ggagtcattg ccttcgactg caggaaccga 780
aaatggtaca tccaggcagc aacttctccg aaagacgtgg tcattttagt tgacgtcagt 840
ggcagcatga aaggactccg tctgactatc gcgaagcaaa cagtctcatc catttggat 900
acacttgggg atgatgactt cttcaacata attgcttata atgaggagct tcactatgtg 960
gaaccttgcc tgaatggaac tttggtgcaa gccgacagga caaacaaaga gcacttcagg 1020
gagcatctgg acaaactttt cgccaaagga attggaatgt tggatatagc tctgaatgag 1080
gccttcaaca ttctgagtga tttcaaccac acgggacaag gaagtatctg cagtcaggcc 1140
atcatgctca taactgatgg ggcggtggac acctatgata caatctttgc aaaatacaat 1200
tggccagatc gaaaggttcg catcttcaca tacctcattg gacgagaggc tgcgtttgca 1260
gacaatctaa agtggatggc ctgtgccaac aaaggatttt ttacccagat ctccaccttg 1320
gctgatgtgc aggagaatgt catggaatac cttcacgtgc ttagccggcc caaagtcatc 1380
gaccaggagc atgatgtggt gtggaccgaa gcttacattg acagcactct ccctcaggca 1440
caaaagctga ctgatgatca gggccccgtc ctgatgacca ctgtagccat gcctgtgttt 1500
agtaagcaga acgaaaccag atcgaagggc attcttctgg gagtggttgg cacagatgtc 1560
ccagtgaaag aacttctgaa gaccatcccc aaatacaagt tagggattca cggttatgcc 1620
tttgcaatca caaataatgg atatatcctg acgcatccgg aactcaggct gctgtacgaa 1680
gaaggaaaaa agcgaaggaa acctaactat agtagcgttg acctctctga ggtggagtgg 1740
gaagaccgag atgacgtgtt gagaaatgct atggtgaatc gaaagacggg gaagttttcc 1800
atggaggtga agaagacagt ggacaaaggg aaacgggttt tggtgatgac aaatgactac 1860
tattatacag acatcaaggg tactcctttc agtttaggtg tggcgctttc cagaggtcat 1920
gggaaatatt tcttccgagg gaatgtaacc atcgaagaag gcctgcatga cttagaacat 1980
cccgatgtgt ccttggcaga tgaatggtcc tactgcaaca ctgacctaca ccctgagcac 2040
cgccatctgt ctcagttaga agcgattaag ctctacctaa aaggcaaaga acctctgctc 2100
cagtgtgata aagaattgat ccaagaagtc ctttttgacg cggtggtgag tgcccccatt 2160
gaagcgtatt ggaccagcct ggccctcaac aaatctgaaa attctgacaa gggcgtggag 2220
gttgccttcc tcggcactcg cacgggcctc tccagaatca acctgtttgt cggggctgag 2280
cagctcacca atcaggactt cctgaaagct ggcgacaagg agaacatttt taacgcagac 2340
catttccctc tctggtaccg aagagccgct gagcagattc cagggagctt cgtctactcg 2400
atcccattca gcactggacc agtcaataaa agcaatgtgg tgacagcaag tacatccatc 2460

cagctcctgg atgaacggaa atctcctgtg gtggcagctg taggcattca gatgaaactt 2520

gaatttttcc aaaggaagtt ctggactgcc agcagacagt gtgcttccct ggatggcaaa 2580

tgctccatca gctgtgatga tgagactgtg aattgttacc tcatagacaa taatggattt 2640

attttggtgt ctgaagacta cacacagact ggagactttt ttggtgagat cgagggagct 2700

gtgatgaaca aattgctaac aatgggctcc tttaaaagaa ttacccttta tgactaccaa 2760

gccatgtgta gagccaacaa ggaaagcagc gatggcgccc atggcctcct ggatccttat 2820

aatgccttcc tctctgcagt aaaatggatc atgacagaac ttgtcttgtt cctggtggaa 2880

tttaacctct gcagttggtg gcactccgat atgacagcta aagcccagaa attgaaacag 2940

accctggagc cttgtgatac tgaatatcca gcattcgtct ctgagcgcac catcaaggag 3000

actacaggga atattgcttg tgaagactgc tccaagtcct ttgtcatcca gcaaatccca 3060

agcagcaacc tgttcatggt ggtggtggac agcagctgcc tctgtgaatc tgtggccccc 3120

atcaccatgg cacccattga aatcaggtat aatgaatccc ttaagtgtga acgtctaaag 3180

gcccagaaga tcagaaggcg cccagaatct tgtcatggct tccatcctga ggagaatgca 3240

agggagtgtg ggggtgcgcc gagtctccaa gcccagacag tcctccttct gctccctctg 3300

cttttgatgc tcttctcaag gtgacactga ctgagatgtt ctcttactga ctgagatgtt 3360

ctcttggcat gctaaatcat ggataaactg tgaaccaaaa tatggtgcaa catacgagac 3420

atgaatatag tccaaccatc agcatctcat catgatttta aactgtgcgt gatataaact 3480

cttaaagata tgttgacaaa aagttatcta tcatcttttt actttgccag tcatgcaaat 3540

gtgagtttgc cacatgataa tcacccttca tcagaaatgg gaccgcaagt ggtaggcagt 3600

gtcccttctg cttgaaacct attgaaacca atttaaaact gtgtactttt taaataaagt 3660

atattaaaat cataaaaaaa aaaaaaaaa 3689

<210> 17
<211> 1166
<212> DNA
<213> Homo sapiens

<400> 17
tcgggtatga ggctgggact aagccaaggg attcaggtgt ggtgccggtg ggaactgagg 60

aagcgcccaa ggaaatgaaa cacgatttcc aaaatgaact taatctttca tgagaaactg 120

aggatagaga tgtcaataag cagccactgt ttccacctcc ccacctgaag agctaggagg 180

acaactacaa agagcctgac tgccttctcg gaatgaggag agaggaaaac agcaacagta 240

tcagttttca agatggcagc atctatgcat ggtcagccca gtccttctct agaagatgca 300

aaactcagaa gaccaatggt catagaaatc atagaaaaaa attttgacta tcttagaaaa 360

gaaatgacac aaaatatata tcaaatggcg acatttggaa caacagctgg tttctctgga 420

atattctcaa acttcctgtt cagacgctgc ttcaaggtta aacatgatgc tttgaagaca 480

tatgcatcat tggctacact tccatttttg tctactgttg ttactgacaa gctttttgta 540

attgatgctt tgtattcaga taatataagc aaggaaaact gtgttttcag aagctcactg 600

attggcatag tttgtggtgt tttctatccc agttctttgg cttttactaa aaatggacgc 660

ctggcaacca agtatcatac cgttccactg ccaccaaaag gaagggtttt aatccattgg 720

atgacgcttt gtcaaacaca aatgaaatta atggcgattc ctctagtctt tcagattatg 780

tttggaatat taaatggtct ataccattat gcagtatttg aagagacact tgagaaaact 840

atacatgaag agtaaccaaa aaaatgaatg gttgctaact tagcaaaatg aagtttctat 900

aaagaggact caggcattgc tgaaagagtt aaaagtaact gtgaacaaat aatttgttct 960

gtgccttttg cctggtatat agcaaatact caaaaaatat tcaataattc aatcaataaa 1020

tataagtttc atcttacacg taagatacag gtcttatctc ctgatggtgt gtccattttg 1080

cctggtatat aacagataat aaatatccag tgtcaataaa aaaaaaaaaa aaaaaaaaaa 1140

aaaaaaaaaa aaaaaaaaaa aaaaaa 1166

<210> 18
<211> 7261
<212> DNA
<213> Homo sapiens

<400> 18
   ctgctccttg caccccgctc cctgcctgga cacaggctca ctcgctgcct tcttctgggg 60
   gaaaccagct tcttgccagc cacagctgct gcctccgcca ctggccaccg cccctgtcct 120
   gggagtccct tggcccagac acccacctga cttagtggct cctctgcagg aaaggtggct 180
   gccccctgcg ttcctccatc caaccatgag ctggtgccca tcaccactga gaatgcacca 240
   aagaatgtag tggacaaggg agaaggagcc tcccggggtg gaaacacacg gaaaagcctc 300
   gaggacaacg gctccaccag ggtcaccccg agtgtccagc cccacctcca gcccatcaga 360
   aacatgagtg tgagccggac catggaggac agctgtgagc tggacctggt gtacgtcaca 420
   gagaggatca tcgctgtctc cttccccagc acagccaatg aggagaactt ccggagcaac 480
   ctccgtgagg tggcgcagat gctcaagtcc aaacatggag gcaactacct gctgttcaac 540
   ctctctgagc ggagacctga catcacgaag ctccatgcca aggtactgga atttggctgg 600
   cccgacctcc acaccccagc cctggagaag atctgcagca tctgtaaggc catggacaca 660
   tggctcaatg cagaccctca caatgtcgtt gttctacaca acaaggaaaa ccgaggcagg 720
   ataggagttg tcatcgcggc ttacatgcac tacagcaaca tttctgccag tgcggaccag 780
   gctctggacc ggtttgcaat gaagcggttc tatgaggata agattgtgcc cattggccag 840
   ccatcccaaa gaaggtacgt gcattacttc agtggcctgc tctccggctc catcaaaatg 900
   aacaacaagc ccttgtttct gcaccacgtg atcatgcacg gcatccccaa ctttgagtct 960
   aaaggaggat gtcggccatt tctccgcatc taccaggcca tgcaacctgt gtacacatct 1020
   ggcatctaca acatcccagg agacagccag actagcgtct gcatcaccat cgagccagga 1080
   ctgctcttga agggagacat cttgctgaag tgctaccaca agaagttccg aagcccagcc 1140
   cgagacgtca tcttccgtgt gcagttccac acctgtgcca tccatgacct gggggttgtc 1200
   tttgggaagg aggaccttga tgatgctttc aaagatgatc gatttccaga gtatggcaaa 1260
   gtggagtttg tattttctta tgggccagag aaaattcaag gcatggagca cctggagaac 1320
   gggccgagcg tgtctgtgga ctataacacc tccgaccccc tcatccgctg ggactcctac 1380
   gacaacttca gtgggcatcg agatgacggc atggaggagg tggtgggaca cacgcagggg 1440
   ccactagatg ggagcctgta tgctaaggtg aagaagaaag actccctgca cggcagcacc 1500
   ggggctgtta atgccacacg tcctacactg tcggccaccc ccaaccacgt ggaacacacg 1560
   ctttctgtga gcagcgactc gggcaactcc acagcctcca ccaagaccga caagaccgac 1620
   gagcctgtcc ccggggcctc cagtgccact gctgccttga gtccccagga gaagcgggag 1680
   ctggaccgcc tgctgagtgg ctttggctta gagcgagaga agcaaggcgc catgtaccac 1740
   acccagcacc tcaggtcccg cccagcaggg ggctcggctg tgccctcctc tggacgccac 1800
   gttgtcccag cccaggttca tgtcaatggt ggggcgttag catctgagcg ggagacagac 1860
   atcctggacg atgaattgcc aaaccaggat ggtcacagtg cgggcagcat gggcacactc 1920
   tcttctctgg acggggtcac caacaccagt gaggggggct acccagaggc cctgtcccca 1980
   ctgaccaacg gtctggacaa gtcctacccc atggagccta tggtcaatgg aggaggctac 2040
   ccctacgagt ctgccagccg ggcggggcct gcccatgctg gccacacggc ccccatgcgg 2100
   ccctcctact ctgcacagga gggtttagct ggctaccaga gggaggggcc ccacccagcc 2160
   tggccacagc cagtgaccac ctcccactat gcccatgacc ccagcggtat gttccgctct 2220
   caatcctttt cggaagctga accccagctg cccccagctc cggtccgagg gggaagcagc 2280
   cgggaggctg tgcaaagggg actgaattcg tggcagcagc agcagcagca gcagcagcag 2340
   cctcgcccac ctccacgcca gcaggaaaga gcccacttgg agagtcttgt agccagcagg 2400
   cccagccctc agccattggc agagaccccc atccccagtc tccctgagtt cccgcgagca 2460
   gcctcccagc aggagattga acagtccatc gaaacactca atatgctgat gctggacctg 2520
   gagccagcct ccgctgctgc cccactacac aagtcccaga gtgtccccgg ggcctggcca 2580
   ggggcttctc cactctcctc ccagcccctc tctggatcct cccgtcagtc ccatccactg 2640
   acccagtcca gatctggcta tatccccagt gggcattcgt tgggaacccc tgagccagcc 2700
   ccacgggcct ctctggagtc tgtccctcct ggcaggtctt actcacctta tgactatcag 2760
   ccatgtttgg ctgggcctaa ccaggatttc cattcaaaga gcccagcctc ttcctccttg 2820
   cctgccttcc ttccgaccac ccacagccct ccagggcctc agcaaccccc agcctctctc 2880
   cctggcctca ctgctcagcc tctgctctca ccaaaggaag cgacttcaga cccctcccgg 2940
   actccagagg aggagccatt gaatttagaa gggctggtgg cccacagggt agcaggggta 3000
   caggctcggg agaagcagcc tgcagagccc ccagcccctc tgcggaggcg ggcggccagt 3060
   gatggacagt atgagaacca gtctccagaa gccacatccc ctcgtagccc tggggttcgc 3120
   tcccctgtcc agtgtgtctc cccggagctg gctcttacca tcgctctcaa tcctggaggg 3180
   cggcccaaag agccccattt gcacagctac aaggaggcct tcgaggagat ggagggaacc 3240
   tccccgagca gcccaccacc cagtggggtg cggtcccccc cgggtctggc caagacaccc 3300
   ctgtctgctc tgggcctgaa acctcacaac ccagcggaca tcctgttgca ccccacagga 3360
   gttaccagaa gacggatcca gccagaggaa gatgagggga aggtggtggt caggctgtca 3420
   gaagagcccc ggagctatgt ggagtctgtg gcacggacag cggtggctgg accccgagct 3480
   caggactctg agcccaagag ctttagtgct ccagccaccc aggcctatgg ccatgagata 3540
   cccctgagga acgggaccct gggtggctcc tttgtctccc ccagccccct ctccaccagc 3600
   agccccatcc tcagtgctga cagcacttca gtggggagtt tcccgtcggg agagagcagt 3660
   gaccagggtc cccggacgcc cacccagcct ctgttggagt ctggcttccg ctcaggcagc 3720
   ctgggacagc ccagcccgtc tgcccagaga aactaccaga gctcttctcc tctcccgact 3780
   gtgggcagta gctacagcag ccccgactac tcacttcagc atttcagctc ctctccggaa 3840
   agccaggctc gagctcagtt cagtgtggct ggcgtccaca cggtgcctgg gagccctcag 3900
   gcgcgccaca gaacagtggg caccaacact ccccctagtc ctggcttcgg ctggcgggcc 3960
   atcaatccca gcatggctgc ccccagcagt cccagtttga gccatcacca gatgatgggt 4020
   ccaccaggca ctggcttcca tggtagcact gtctccagcc cccagagcag tgcagcgacc 4080
   accccgggga gccccagcct gtgtcggcac ccagcagggg tctaccaggt ttctggcctc 4140
   cacaacaaag tggccaccac cccggggagt cccagcctgg gccggcaccc tggggctcac 4200
   caaggcaacc tggcctccgg tcttcatagc aatgcaatag ccagccctgg aagccccagc 4260
   ctgggccgtc acctcggagg gtctggatct gtggttcccg gcagcccctg cttggaccgg 4320
   catgtggcct atggtggcta ttctaccccg gaggatcgga gacccacact gtcccggcag 4380
   agcagtgcct ctggctacca ggctccttcc acgccctcct tccctgtctc ccctgcctac 4440
   taccctggcc tgagcagccc tgccacctcc ccgtcaccag actccgcagc cttccggcaa 4500
   gggagcccaa caccagcctt gccagagaag cgaaggatgt cagtgggaga ccgggcaggc 4560
   agcctcccca actatgccac catcaatggg aaggtgtctt cgcctgtcgc cagcggcatg 4620
   tccagtccca gtgggggcag caccgtctcc ttctcccaca ctctgcccga cttctccaag 4680
   tactccatgc cagacaacag cccggagacg cgggctaaag tgaagtttgt ccaggacact 4740
   tctaagtatt ggtacaagcc tgagatctcc agggagcagg ccatcgcgct cctcaaggac 4800
   caggagccgg gggccttcat catccgcgac agtcactcct tccgaggcgc gtacgggctg 4860
   gccatgaagg tgtcttcgcc acctccaacc atcatgcagc agaataaaaa aggagacatg 4920
   acccatgagc tggtcaggca ttttctgata gagactggcc ccagaggagt caagctcaag 4980
   ggctgcccca atgagccaaa cttcggatcg ctgtctgccc tggtctacca gcactccatc 5040
   atcccattgg ccctgccttg caagctggtc attccaaacc gagaccccac agatgaatcg 5100
   aaagatagct ccggccctgc caactcaact gcagacctgc tgaaacaagg ggcagcctgc 5160
   aatgtgctct tcgtcaactc tgtggacatg gagtcactca ctgggccaca ggccatctct 5220
   aaagccacat ctgagacgtt ggctgcagac cccacgccag ctgccaccat cgttcacttc 5280
   aaagtctctg cccagggaat cactctgact gacaaccaga gaaagctctt tttcagacgc 5340
   cactaccctc tcaacactgt caccttctgt gacctggatc cacaggaaag aaagtggatg 5400
   aaaacagagg gtggtgcccc tgctaagctc ttcggcttcg tggcccggaa gcagggcagc 5460
   accacggaca acgcctgcca cctctttgct gagcttgacc ccaaccagcc ggcctctgcc 5520
   atcgtcaact tcgtctccaa ggtcatgctg aatgccggcc aaaagagatg aaccctgccc 5580
   cttgcccagg gccagtgcca tggggaaggg gcttgtgggg aggggaccca tgaatcctga 5640
   ccactcttga acccagaagg aggactttgg gccaatttcg gaggagagaa gaaagtgcaa 5700
   cgtggggaga gggaagtgaa ttgcagaggg gagggggaaa agagagagag agagagagag 5760
   agagagagag agaaagatgg aggagaagaa cttggattcc cctgggtaga tggaaactgc 5820
   aaaaacccaa agcctccaaa actaaccagg tccacctaac accccctccc tcccctaaga 5880
   agatggatgt cctcaaaaga gaaggaacaa acctccttgg gaatccacat tttttggggg 5940
   aatggaaaag ctctgtctcc ctaactcaac tgctttgcaa ggggaaatca agctgggaga 6000
   atctttttct ggccacctgt ggggtaggtt gtcaaaccaa acagagccac cgtgggacat 6060
   caagtggaag aacttgtttg cttgaaagta tctcagaccc aaggcacctc aggtctcttt 6120
   gctgtgcctc cactatattg tcgtgtgggt gtgtgtctgc acccacatcc tcacacattg 6180
   atctagatct gcctttatcc actcgaatta taaacagctc ggcttgtcct tgtcccatgt 6240
   gtttgtagac acacatgcat actgtccaaa gattagggtt ggtggtggca gtgcagcagg 6300
   ggagggacaa acaaccaagc tatgggtgac agaggctctc tcctggtgcc tgcacctgca 6360
   ctctagtgac cctgggtgcc gccagaccct tctcttctac aaagacccca gcaggagtgg 6420
   gagggtctgc aatggcatcg ccctgtcctg ccttggccag aagcctggag ctttggtttg 6480
   aggaggtaga gatatgtgta tccataggaa gagatctgtc agaacaggca gctgttgagc 6540
   tcggggtgtc ttccccaagg catgtggctc agcagcaaga aaggcaagtt gctcctgctg 6600
   gggccctgga ctctgcctta gctcccacct ctcagccttg ttattgggtt tcatgcccct 6660
   ggaccagcct tatctcagac ctgcttacct gcatgatgcc tttttggggg ctggggattg 6720
   agtcttgctg ctctgcccag ccctgttcta ttctgcaggg tccctgtgtt ggaattctcc 6780
   ctggggaacc tactttctgc tcagtgaggc tccggccaga aacctggagt ccttatcctc 6840
   ccctctgtaa gtgttttagg gtctggcttt tgcaggcacc ctctgacctc agcagagctc 6900
   ctgggcctgc tgcctgcaca ccacatcgcc tacctacaat gccaaagcct cactgtcacc 6960
   ctttctgcct tggtttccct agctgagcca cgctgcccat gcagcagagg gcagaaggct 7020
   tgcacttggg ccaaagggcc taaggtccac tggacagttg ggaaaacacc tgaccaccat 7080
   ttaaggactc taagccagaa tggaaaattc accaggactc cattcttaag cctatgcgag 7140
   tcccctagag agaggcattg tactgatata taaatattat ataatatata catgagacat 7200
   actgacagaa tctgtaagct aataaaatgt aagaaaaggt taaaaaaaga aaaaaaaaaa 7260
   a 7261

<210> 19
<211> 2500
<212> DNA
<213> Homo sapiens

<400> 19
cccaggcgca gccaatggga agggtcggag gcatggcaca gccaatggga agggccgggg 60

caccaaagcc aatgggaagg gccgggagcg cgcggcgcgg gagatttaaa ggctgctgga 120

gtgaggggtc gcccgtgcac cctgtcccag ccgtcctgtc ctggctgctc gctctgcttc 180

gctgcgcctc cactatgctc tccctccgtg tcccgctcgc gcccatcacg gacccgcagc 240

agctgcagct ctcgccgctg aaggggctca gcttggtcga caaggagaac acgccgccgg 300

ccctgagcgg gacccgcgtc ctggccagca agaccgcgag gaggatcttc caggagccca 360

cggagccgaa aactaaagca gctgcccccg gcgtggagga tgagccgctg ctgagagaaa 420

acccccgccg ctttgtcatc ttccccatcg agtaccatga tatctggcag atgtataaga 480

aggcagaggc ttccttttgg accgccgagg aggttgacct ctccaaggac attcagcact 540

gggaatccct gaaacccgag gagagatatt ttatatccca tgttctggct ttctttgcag 600

caagcgatgg catagtaaat gaaaacttgg tggagcgatt tagccaagaa gttcagatta 660

cagaagcccg ctgtttctat ggcttccaaa ttgccatgga aaacatacat tctgaaatgt 720

atagtcttct tattgacact tacataaaag atcccaaaga aagggaattt ctcttcaatg 780

ccattgaaac gatgccttgt gtcaagaaga aggcagactg ggccttgcgc tggattgggg 840

acaaagaggc tacctatggt gaacgtgttg tagcctttgc tgcagtggaa ggcattttct 900

tttccggttc ttttgcgtcg atattctggc tcaagaaacg aggactgatg cctggcctca 960

catttctaa tgaacttatt agcagagatg agggtttaca ctgtgatttt gcttgcctga 1020

tgttcaaaca cctggtacac aaaccatcgg aggagagagt aagagaaata attatcaatg 1080

ctgttcggat agaacaggag ttcctcactg aggccttgcc tgtgaagctc attgggatga 1140

attgcactct aatgaagcaa tacattgagt ttgtggcaga cagacttatg ctggaactgg 1200

gttttagcaa ggttttcaga gtagagaacc catttgactt tatggagaat atttcactgg 1260

aaggaaagac taacttcttt gagaagagag taggcgagta tcagaggatg ggagtgatgt 1320

caagtccaac agagaattct tttaccttgg atgctgactt ctaaatgaac tgaagatgtg 1380

cccttacttg gctgattttt tttttccatc tcataagaaa aatcagctga agtgttacca 1440

actagccaca ccatgaattg tccgtaatgt tcattaacag catctttaaa actgtgtagc 1500

tacctcacaa ccagtcctgt ctgtttatag tgctggtagt atcacctttt gccagaaggc 1560

ctggctggct gtgacttacc atagcagtga caatggcagt cttggcttta aagtgagggg 1620

tgacccttta gtgagcttag cacagcggga ttaaacagtc ctttaaccag cacagccagt 1680

taaaagatgc agcctcactg cttcaacgca gattttaatg tttacttaaa tataaacctg 1740

gcactttaca aacaaataaa cattgttttg tactcacggc ggcgataata gcttgattta 1800

tttggtttct acaccaaata cattctcctg accactaatg ggagccaatt cacaattcac 1860

taagtgacta aagtaagtta aacttgtgta gactaagcat gtaattttta agttttattt 1920

taatgaatta aaatatttgt taaccaactt taaagtcagt cctgtgtata cctagatatt 1980

agtcagttgg tgccagatag aagacaggtt gtgtttttat cctgtggctt gtgtagtgtc 2040

ctgggattct ctgccccctc tgagtagagt gttgtgggat aaaggaatct ctcagggcaa 2100

ggagcttctt aagttaaatc actagaaatt taggggtgat ctgggccttc atatgtgtga 2160

gaagccgttt cattttattt ctcactgtat tttcctcaac gtctggttga tgagaaaaaa 2220

ttcttgaaga gttttcatat gtgggagcta aggtagtatt gtaaaatttc aagtcatcct 2280

taaacaaaat gatccaccta agatcttgcc cctgttaagt ggtgaaatca actagaggtg 2340

gttcctacaa gttgttcatt ctagttttgt ttggtgtaag taggttgtgt gagttaattc 2400

atttatattt actatgtctg ttaaatcaga aatttttat tatctatgtt cttctagatt 2460

ttacctgtag ttcataaaaa aaaaaaaaaa aaaaaaaaaa 2500

<210> 20
<211> 67
<212> DNA
<213> Homo sapiens

<400> 20
accgcccgta acgcgacatg atgacagacc cgcgtgctcg gctctttagc aagcagcgcc 60

caggggct                                                          67


<210>  21
<211>  54
<212>  DNA
<213>  Homo sapiens

<400>  21
acagccatgt ttcccagctg gggcacacca gggccagccc ctggcatggc ctgt          54


<210>  22
<211>  70
<212>  DNA
<213>  Homo sapiens

<400>  22
tgggcctggt gttggtggca tccatggctg atctggaggg gggtgtgggg gttgctgatg    60

cattccccat                                                           70


<210>  23
<211>  61
<212>  DNA
<213>  Homo sapiens

<400>  23
atggcctgag ccgagtatca agagcgacag caccaccggc gccggcggta gcagcggggc    60

t                                                                    61


<210>  24
<211>  52
<212>  DNA
<213>  Homo sapiens

<400>  24
aggctgccac agaggaccac gcagggggtt gcacctgagg actgggtacc ac           52


<210>  25
<211>  69
<212>  DNA
<213>  Homo sapiens

<400>  25
acctgcagcg cagccggcgc ggtccagtga aagcgcctct tcagcagccc ggagacctcc    60

cgaaggggt                                                            69


<210>  26
<211>  64
<212>  DNA
<213>  Homo sapiens

<400>  26
agctggcgca tgtgcttgac attgcgctgg tgggctcgcc tcagggcttg gccctcctct    60

ccct                                                                 64

<210>  27
<211>  61
<212>  DNA
<213>  Homo sapiens

<400>  27
acggccggct cgtccgctcg gaaagctctg ggatgctcgg gcgcagtgac agccgcgctc      60

t                                                                      61


<210>  28
<211>  69
<212>  DNA
<213>  Homo sapiens

<400>  28
agcgcaggca caggaagctg gtgacggcga tgaacggtac attctcgtgc tgggcgccgg      60

ggaagcgct                                                              69


<210>  29
<211>  68
<212>  DNA
<213>  Homo sapiens

<400>  29
agccggaggc atgacaaacc agcaacagct ggccagaccc aaggctgggg cgactggaca      60

gccaggct                                                               68


<210>  30
<211>  64
<212>  DNA
<213>  Homo sapiens

<400>  30
cggcggcgtc cacgggcaga aaggagcaag ctgacgaacg tatctgccag cctaccttcg      60

ccct                                                                   64


<210>  31
<211>  70
<212>  DNA
<213>  Homo sapiens

<400>  31
tggcacgcgc tcgtgggtcc accgccgggc agaaccgaaa ctttctgggg ccaagcggaa      60

gaggacggga                                                             70


<210>  32
<211>  55
<212>  DNA
<213>  Homo sapiens

<400>  32

accgctttcc cgctccccgt gcggctccct atggctgctg gacagcaccg gagcg 55

<210> 33
<211> 66
<212> DNA
<213> Homo sapiens

<400> 33
agggccgcag ccatgtttgc gccgagtgaa cgcattgcct gggcagcctc ggcccgagtc 60

cgggct 66

<210> 34
<211> 65
<212> DNA
<213> Homo sapiens

<400> 34
acgctggcga gactgtcgat cgcgccgggg ggcgaggggc tggggtcccg ctttacatct 60

ggggt 65

<210> 35
<211> 51
<212> DNA
<213> Homo sapiens

<400> 35
agggccaggc tggtccaata cgcttcaatg ggggcactca ccaccgcgtc a 51

<210> 36
<211> 63
<212> DNA
<213> Homo sapiens

<400> 36
ccttgggcgc ttcctcagtt cccaccggca ccacacctga atcccttggc ttagtcccag 60

cct 63

<210> 37
<211> 70
<212> DNA
<213> Homo sapiens

<400> 37
atggccagcc cgtacgcgcc tcggaaggag tgactgtcgc ggatgatgaa ggcccccggc 60

tcctggtcct 70

<210> 38
<211> 68
<212> DNA
<213> Homo sapiens

<400> 38
acgcgggtcc cgctcagggc cggcggcgtg ttctccttgt cgaccaagct gagccccttc 60

agcggcga                                                              68

<210>  39
<211>  22
<212>  DNA
<213>  Artificial

<220>
<223>  primer sequence

<400>  39
acccacattt tcagaccata tg                                              22

<210>  40
<211>  22
<212>  DNA
<213>  Artificial

<220>
<223>  primer sequence

<400>  40
actgaagctc tcaagttcac cc                                              22

<210>  41
<211>  21
<212>  DNA
<213>  Artificial

<220>
<223>  primer sequence

<400>  41
ttctcatgtg aggctcttgt g                                               21

<210>  42
<211>  19
<212>  DNA
<213>  Artificial

<220>
<223>  primer sequence

<400>  42
ttgctgtgtc cctctctgc                                                  19

<210>  43
<211>  21
<212>  DNA
<213>  Artificial

<220>
<223>  primer sequence

<400>  43
attaaccaca tcaccgtctg c                                               21

<210> 44
<211> 21
<212> DNA
<213> Artificial

<220>
<223> primer sequence

<400> 44
aagaattacg gcatcgaatc c 21

<210> 45
<211> 22
<212> DNA
<213> Artificial

<220>
<223> primer sequence

<400> 45
atcatgctgt gagttttcat gg 22

<210> 46
<211> 22
<212> DNA
<213> Artificial

<220>
<223> primer sequence

<400> 46
aacccaaaag aaagaaaaag gg 22

<210> 47
<211> 18
<212> DNA
<213> Artificial

<220>
<223> primer sequence

<400> 47
tgtgccagct gctctgag 18

<210> 48
<211> 21
<212> DNA
<213> Artificial

<220>
<223> primer sequence

<400> 48
ttttgatgaa ggttggtgag g 21

<210> 49
<211> 22

<212>  DNA
<213>  Artificial

<220>
<223>  primer sequence

<400>  49
gacactggct tctatggttt cc                                                22


<210>  50
<211>  21
<212>  DNA
<213>  Artificial

<220>
<223>  primer sequence

<400>  50
gttccttttg aatttctccc g                                                 21


<210>  51
<211>  18
<212>  DNA
<213>  Artificial

<220>
<223>  primer sequence

<400>  51
catccacgaa ggtcaggg                                                     18


<210>  52
<211>  19
<212>  DNA
<213>  Artificial

<220>
<223>  primer sequence

<400>  52
tccgtaacag tggagcagc                                                    19


<210>  53
<211>  18
<212>  DNA
<213>  Artificial

<220>
<223>  primer sequence

<400>  53
cccaggtcaa gatgcacc                                                     18


<210>  54
<211>  20
<212>  DNA
<213>  Artificial

<220>
<223>  primer sequence

<400>  54
ctcggagacc ttcacaaagc                                                    20


<210>  55
<211>  20
<212>  DNA
<213>  Artificial

<220>
<223>  primer sequence

<400>  55
gcctggacag taagagcagg                                                    20


<210>  56
<211>  19
<212>  DNA
<213>  Artificial

<220>
<223>  primer sequence

<400>  56
acgctgtagc catcactgc                                                     19


<210>  57
<211>  18
<212>  DNA
<213>  Artificial

<220>
<223>  primer sequence

<400>  57
cctgttcatt ccgcatcc                                                      18


<210>  58
<211>  20
<212>  DNA
<213>  Artificial

<220>
<223>  primer sequence

<400>  58
tgaaggcgtc acagaaacag                                                    20


<210>  59
<211>  20
<212>  DNA
<213>  Artificial

<220>
<223>  primer sequence

<400>  59
ttgtagcttc caccacctcc                                                    20


<210>  60
<211>  21
<212>  DNA
<213>  Artificial

<220>
<223>  primer sequence

<400>  60
caatgatggt ggttatggag g                                                  21


<210>  61
<211>  21
<212>  DNA
<213>  Artificial

<220>
<223>  primer sequence

<400>  61
cactgaagga gatgctcttg g                                                  21


<210>  62
<211>  20
<212>  DNA
<213>  Artificial

<220>
<223>  primer sequence

<400>  62
aggacactga agacatgccc                                                    20


<210>  63
<211>  21
<212>  DNA
<213>  Artificial

<220>
<223>  primer sequence

<400>  63
tgttctcatg gaagctatgg g                                                  21


<210>  64
<211>  18
<212>  DNA
<213>  Artificial

<220>
<223>  primer sequence

<400>  64
ccgacatgtc ttctgggg                                                      18

<210> 65
<211> 18
<212> DNA
<213> Artificial

<220>
<223> primer sequence

<400> 65
ccaatgaatg gtggcctc 18

<210> 66
<211> 20
<212> DNA
<213> Artificial

<220>
<223> primer sequence

<400> 66
ttggctttat tgcccaaaac 20

<210> 67
<211> 19
<212> DNA
<213> Artificial

<220>
<223> primer sequence

<400> 67
agtgtggatg gggaggaag 19

<210> 68
<211> 22
<212> DNA
<213> Artificial

<220>
<223> primer sequence

<400> 68
ccactagaga tcccataggt gc 22

<210> 69
<211> 18
<212> DNA
<213> Artificial

<220>
<223> primer sequence

<400> 69
agacagatgg cggtgctc 18

<210> 70
<211> 20

<212>  DNA
<213>  Artificial

<220>
<223>  primer sequence

<400>  70
acagtggaca aagggaaacg                                                    20


<210>  71
<211>  20
<212>  DNA
<213>  Artificial

<220>
<223>  primer sequence

<400>  71
tgccaatcag tgagcttctg                                                    20


<210>  72
<211>  19
<212>  DNA
<213>  Artificial

<220>
<223>  primer sequence

<400>  72
tggcgacatt tggaacaac                                                     19


<210>  73
<211>  18
<212>  DNA
<213>  Artificial

<220>
<223>  primer sequence

<400>  73
tgagcggaag ccagactc                                                      18


<210>  74
<211>  20
<212>  DNA
<213>  Artificial

<220>
<223>  primer sequence

<400>  74
caggactctg agcccaagag                                                    20


<210>  75
<211>  20
<212>  DNA
<213>  Artificial

<220>
<223>  primer sequence

<400>  75
tcccatcctc tgatactcgc                                                    20


<210>  76
<211>  20
<212>  DNA
<213>  Artificial

<220>
<223>  primer sequence

<400>  76
caggagttcc tcactgaggc                                                    20